(19) Europäisches Patentamt / European Patent Office / Office européen des brevets

(11) **EP 4 442 212 B1**

(12) **EUROPEAN PATENT SPECIFICATION**

(45) Date of publication and mention
of the grant of the patent:
**22.07.2026  Bulletin 2026/30**

(21) Application number: **24190041.4**

(22) Date of filing: **11.02.2020**

(51) International Patent Classification (IPC):
*A61B 17/221* (2006.01)     *A61B 17/22* (2006.01)

(52) Cooperative Patent Classification (CPC):
**A61B 17/221; A61B 17/22031;** A61B 2017/2215

(54) **ANTI-LOCKING THROMBECTOMY APPARATUSES**

ANTI-LOCK-THROMBEKTOMIEVORRICHTUNGEN

APPAREILS ANTI-BLOCAGE POUR THROMBECTOMIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**09.10.2024  Bulletin 2024/41**

(62) Document number(s) of the earlier application(s) in
accordance with Art. 76 EPC:
**20710389.6 / 4 103 076**

(73) Proprietor: **Stryker Corporation
Fremont, CA 94538 (US)**

(72) Inventors:
• **WALLACE, Michael P.**
**Pleasanton, CA 94566 (US)**
• **DELOS SANTOS, Jayson**
**Fremont, CA 94536 (US)**
• **GREENHALGH, E. Skott**
**Gladwyne, PA 19035 (US)**
• **TANG, Winnie**
**Santa Clara, CA 95051 (US)**
• **VAN, Clifford**
**Santa Clara, CA 95051 (US)**

(74) Representative: **Vossius & Partner
Patentanwälte Rechtsanwälte mbB
Siebertstrasse 3
81675 München (DE)**

(56) References cited:
WO-A2-2012/009675     US-A1- 2017 303 948
US-A1- 2019 133 624

## Description

### FIELD

The apparatuses and methods described herein relate to mechanical removal of material from within a body lumen. For example, described herein are mechanical thrombectomy apparatuses and methods.

### BACKGROUND

It is often desirable to remove tissue from the body in a minimally invasive manner as possible, so as not to damage other tissues. For example, removal of tissue from within a vasculature, such as blood clots, may improve patient conditions and quality of life.

Many vascular system problems stem from insufficient blood flow through blood vessels. One causes of insufficient or irregular blood flow is a blockage within a blood vessel referred to as a blood clot, or thrombus. Thrombi can occur for many reasons, including after a trauma such as surgery, or due to other causes. For example, a large percentage of the more than 1.2 million heart attacks in the United States are caused by blood clots (thrombi) which form within a coronary artery.

When a thrombus forms, it may effectively stop the flow of blood through the zone of formation. If the thrombus extends across the interior diameter of an artery, it may cut off the flow of blood through the artery. If one of the coronary arteries is 100% thrombosed, the flow of blood is stopped in that artery, resulting in a shortage of oxygen carrying red blood cells, e.g., to supply the muscle (myocardium) of the heart wall. Such a thrombosis is unnecessary to prevent loss of blood but can be undesirably triggered within an artery by damage to the arterial wall from atherosclerotic disease. Thus, the underlying disease of atherosclerosis may not cause acute oxygen deficiency (ischemia) but can trigger acute ischemia via induced thrombosis. Similarly, thrombosis of one of the carotid arteries can lead to stroke because of insufficient oxygen supply to vital nerve centers in the cranium. Oxygen deficiency reduces or prohibits muscular activity, can cause chest pain (angina pectoris), and can lead to death of myocardium which permanently disables the heart to some extent. If the myocardial cell death is extensive, the heart will be unable to pump sufficient blood to supply the body's life sustaining needs. The extent of ischemia is affected by many factors, including the existence of collateral blood vessels and flow which can provide the necessary oxygen.

Clinical data indicates that clot removal may be beneficial or even necessary to improve outcomes. For example, in the peripheral vasculature, inventions and procedures can reduce the need for an amputation by 80 percent. The ultimate goal of any modality to treat these conditions of the arterial or venous system is to remove the blockage or restore patency, quickly, safely, and cost effectively. This may be achieved by thrombus dissolution, fragmentation, thrombus aspiration or a combination of these methods.

Existing device for removing material (including clots) from within a body lumen often face difficulty with removing a large amount material, and/or hard or rigid material. Mechanical device for removing such materials may jam or clog. Further, when mechanical devices are used, they may lock up.

Examples of devices that include an inverting tube for removing material from a body lumen, such as for removing a clot from a blood vessel (e.g., thrombectomy devices), are disclosed and described in each of U.S. Patent No. 10,271,864, as well as in each of U.S. Patent Application Publication Nos. 2017/0303948, 2019/0117214, 2018/0042626 and 2018/0042624, and in U.S. Patent Application Serial No. 16/566,393. These apparatuses do an excellent job at removing material from within a blood vessel, but in some situations may face challenges when using longer inverting tubes, and in particular, when using knitted inverting tubes that fit snugly over the inversion support catheter, for example, when constrained within a delivery catheter or in other low-profile situations. In some cases, the knitted tractor portion may jam or lock onto the outside of the inversion support catheter when removing material. This problem may be particularly acute when removing material from tortious vessels. Thus, there is a need for devices, including thrombectomy devices, that can remove tissue, and particularly large and/or hard materials, from within a body lumen without jamming or locking up. Described herein are apparatuses (devices, systems and kit) and methods of using them that may address the needs and problems discussed above.

### SUMMARY OF THE INVENTION

The invention is directed to an apparatus for removing a material from a vessel as defined in claim 1. Embodiments of the invention are recited in the dependent claims.

### SUMMARY OF THE DISCLOSURE

Described herein are mechanical apparatuses (devices, systems, etc.) and methods of using and making them.

For example, described herein are mechanical thrombectomy apparatuses. These apparatuses may be configured to prevent or reduce jamming particularly when removing large amounts of material, or hard/rigid materials. The mechanical thrombectomy apparatuses described herein include an inverting tube (also referred to herein as an inverting tractor, a tractor, a tractor region, a tractor portion, etc.) comprising a flexible tube of material that inverts over itself as it rolls over a distal end opening of an elongate inversion support (also referred to herein as an inversion support catheter). The tractor may be a knitted tube having a plurality of interlocking loops (e.g., links). The inversion support typically includes a catheter having a distal end opening into which the knitted tube inverts. The flexible knitted tube inverts and rolls back into itself and may be drawn into the elongate inversion support in a conveyor-like motion; the outward-facing region rolls around to become an inward-facing region, e.g., within the lumen of the elongate inversion support. The rolling motion may thus draw a clot or other material within a body lumen into the elongate inversion support, so that it may be removed from the body lumen.

In some variations, the methods and apparatuses described herein may address these situations and may prevent locking down of the knitted tube onto the outside of the inversion support catheter. For example, described herein are apparatuses and methods for removing a material from a body lumen including a knitted tube portion that is configured so that, even when the knitted tube is held in close proximity to the outer surface of the inversion support catheter (e.g., within 1 mm, within 0.8 mm, within 0.5 mm, within 0.4 mm, within 0.2 mm, etc. on average), the knitted tube does not change diameter and lock onto the outer surface of the inversion support catheter when pulled (e.g., to roll and invert into the inversion support catheter). For example, the knitted tube may be configured to ride over the outer surface of the catheter in the un-inverted configuration within a distance of about 0.5 mm. The knitted tube may be configured so that it does not stretch (and therefore constrict) more than a predefined percentage when pulled in tension. For example, the knitted tube may be configured so that it does not stretch more than 3 percent when pulled in tension with a force of 2 Newtons. In general, the knitted tube may be configured so as not to stretch while still remaining sufficiently flexible so as to roll over the distal end of the catheter easily, capture clot, and not jam within the distal end opening of the inversion support catheter.

Thus, described herein are particular configurations of the knitted tractor, such as the dimensions of the knitted filament forming the knitted tube (e.g., filament width), the number of loops of the knitted tube per turn, and the size (e.g., circumference) of the inversion support catheter over which the knitted tube is to be pulled. The material forming the knitted tube (e.g., stainless steel, Nitinol, etc.) may also contribute to the functioning of the device. In some variations the size of the loops (e.g., the length of the loops) may also contribute. Without being bound by a particular theory of operation, described herein are methods and apparatuses in which the relationship between the number of loops per turn, the circumference of the inversion support catheter and the thickness (e.g., cross-sectional thickness) of the filament forming the loops of the knitted tube may define a range of values that have been found empirically, as described herein, to provide a knitted tube that resists locking down on to the inversion support catheter. Surprisingly, outside of the specified range, the knitted tubes may otherwise lock onto the inversion support catheter.

For example, described herein are apparatuses for removing a material from a body lumen, the apparatus comprising: an elongate inversion support comprising a catheter having a circumference, a distal end, and a distal end opening; a knitted tube that extends distally in an un-inverted configuration along an outer surface of the catheter, inverts over the distal end opening of the catheter and extends proximally within the catheter in an inverted configuration, wherein the knitted tube is configured to invert by rolling over the distal end opening of the catheter when a first end of the knitted tube is pulled proximally within the catheter; further wherein the knitted tube is configured to stretch less than 3 percent when pulled in tension with a force of 2 Newtons, so that the knitted tube does not lock onto the outer surface of the catheter. In any of these apparatuses, the knitted tube may be configured to be, in the un-inverted configuration on the outer surface of the catheter, spaced (at rest) about 1 mm or less, on average, from the outer surface of the catheter.

For example, an apparatus for removing a material from a body lumen may include: an elongate inversion support comprising a catheter having a circumference, a distal end, and a distal end opening; a knitted tube that extends distally in an un-inverted configuration along an outer surface of the catheter, inverts over the distal end opening of the catheter and extends proximally within the catheter in an inverted configuration, wherein the knitted tube is configured to invert by rolling over the distal end opening of the catheter when a first end of the knitted tube is pulled proximally within the catheter; further wherein the knitted tube has a length of 20 cm or greater and comprises a plurality of N loops per turn formed of a filament having a filament diameter and wherein the knitted tube has a ratio of a square of N loops per turn multiplied by the filament diameter, per the circumference of the catheter that is greater than 2.9.

The number of loops of the knitted tube per turn may be referred to as the number of needles forming the knitted tube, and refers to the number of loops in a full circumference of the knitted tube.

In some variations, the knitted tube is configured to stretch less than 2 percent when pulled in tension with a force of 2 Newtons. As mentioned above, the knitted tube may be formed of a filament that is knitted into a number of N loops per turn, and wherein the knitted tube is configured to stretch less than 3 percent when pulled in tension with a force of 2 Newtons based on the number of N loops per turn and a diameter of the filament, so that the knitted tube does not lock onto the outer surface of the catheter. In some variations the knitted tube has a length of 20 cm or greater and comprises a number of N loops per turn formed of a filament having a filament diameter and wherein the ratio of a square of N loops per turn multiplied by the filament diameter, per the circumference of the catheter that is greater than 2.9. For example, the ratio of the square of N loops per the circumference of the catheter per the filament diameter may be greater than 3.0.

In any of these apparatuses, the knitted tube may have a length of 65 cm or greater (e.g., 70 cm or longer, 75 cm or longer, 80 cm or longer, 85 cm or longer, 90 cm or longer, 100 cm or longer, 110 cm or longer, 120 cm or longer, 130 cm or longer, 140 cm or longer, 150 cm or longer, etc.).

The filament may be a wire, and may have any appropriate filament diameter, e.g., the filament may have a filament diameter of between 0.02 mm and 0.07 mm (e.g., between about 0.03 and 0.06). In some variations, the catheter may have a circumference of between 3 mm and 13 mm, e.g., between 4 mm and 12 mm, etc.

Any of the apparatuses described herein may include a puller within the catheter, wherein the first end of the knitted tube is coupled to the puller so that pulling the puller proximally pulls the knitted tube proximally within the catheter and rolls and inverts the knitted tube. The elongate puller may be, for example, a hypotube having an inner lumen that is continuous with a lumen though the knitted tube.

In general, any of these apparatuses may be configured so that the knitted tube is biased to expand to greater than the inner diameter of the catheter in the inverted configuration. The knitted tube may also or alternatively be biased to have an inner diameter in the un-inverted configuration that is just slightly larger (e.g., between 0.1 mm and 3 mm, between 0.1 mm and 2 mm, between 0.1 mm and 1 mm, 1.2 mm or less, 1 mm or less, 0.8 mm or less, 0.6 mm or less, etc.) than the outer diameter of the catheter.

Any of these apparatuses may include a guidewire lumen extending through the knitted tube and configured to pass a guidewire.

The filament may be any appropriate material. For example, the filament may comprise one or more of: steel, polyester, nylon, expanded Polytetrafluoroethylene (ePTFE), or Nitinol.

The knitted tube may comprise one or more coatings from the group of: a lubricious coating, a metal coating, a heparin coating, an adhesive coating, and a drug coating.

Also described herein are methods of using any of these apparatuses. For example, described herein are methods for removing material from within a body lumen that include: positioning a distal end of an elongate inversion support comprising a catheter adjacent to the material; pulling a first end of a knitted tube so that the knitted tube is drawn proximally into the catheter by rolling over a distal end opening of catheter and inverting from an un-inverted configuration along an outer surface of the catheter, into an inverted configuration within the catheter, wherein the knitted tube is configured to stretch less than 3 percent when pulled in tension with a force of 2 Newtons so that the knitted tube does not lock onto the out surface of the catheter; and capturing the material with the knitted tube and drawing the material into the catheter. As mentioned, the knitted tube may have a length of 20 cm or greater and may comprise a plurality of N loops per turn formed of a filament having a filament diameter and wherein the knitted tube has a ratio of a square of N loops per turn multiplied by the filament diameter, per the circumference of the catheter that is greater than 2.9. In some variations, pulling the first end of the knitted tube comprises pulling proximally on a puller within the catheter, wherein the puller is attached to the first end of the knitted tube.

Any of these apparatuses may include an expandable funnel on the end of an inversion support catheter over which a tractor (in some variations, a knitted tube) rolls to invert. The tractor may have a first end coupled at a distal end region of a puller (which may be an elongate wire, tube, cannula, etc.), and the flexible tube may be arranged to invert over a distal end of the inversion support catheter at the funnel so that an external portion of the flexible tube extends proximally over the inversion support catheter as the internal (inverted) portion of the flexible tube is drawn into the funnel, compressing and removing fluid from the clot that is held by (e.g., grabbed by) the flexible tube, and pulling the inverted flexible tube into the inversion support catheter until the entire clot is captured, compressed and drawn into the outer catheter. The configuration of the apparatuses described herein are particularly well suited to grabbing and removing clot, and particularly large diameter clots, by using an inversion support catheter than includes an expandable funnel at the distal end. The collapsible/expandable funnel may be configured to operate with the compressive forces applied by pulling the flexible tube into the inversion support catheter so that it inverts into the inversion support catheter, capturing the clot. The collapsible/expandable funnel may be configured to assume a fully expanded, locked (e.g., "jammed") configuration when the flexible tube applies a laterally compressive force on the distal end face of the funnel. Further, the funnel may include openings (described herein as having a porous structure) through which fluid squeezed out of the clot may exit laterally out of the funnel as the clot is moved into the narrower-diameter lumen of the inversion support catheter and compresses the clot. For example, the openings through the collapsible/expandable funnel (which may be referred to herein as simply an expandable funnel) that permit fluid to laterally leave the funnel walls as the clot is compressed may also prevent clogging or jamming of the clot.

The interior of the funnels described herein may be shaped so that the material drawn into the funnel is held up within the funnel, allowing it to be broken down and compressed within the funnel without causing the tractor to jam, preventing pulling in the tractor and/or additional clot. Thus, described herein are methods and apparatuses in which the distal end region of the inversion support catheter is configured as a collapsible and/or expandable funnel. The expandable funnel apparatuses described herein may also or alternatively be adapted to prevent jamming of the tractor in the distal end opening of the funnel, including when the apparatus is used to remove large and/or hard clots. In some variations these funnels may be adapted to include an inner wall within the funnel that divides the funnel into section, such as a small section (e.g., 30% or less, 25% or less, 20% or less, 15% or less, 10% or less, etc.) of the proximal (narrower) end of the funnel may have a steeper angle relative to the more distal end. In some variations the funnel inner lumen includes two or more chambers that are separated by a narrowing or constricted region. These adaptations to the inner lumen of the funnel may permit harder clots to be retained within the funnel as the tractor is pulled into and past the held-up clot, helping break even the harder clots down.

Alternatively, or additionally, in some variations the clot may be formed at least in part by a plurality of tines, arms, etc. formed (e.g., by cutting, etc.) into the distal end region of the inversion support catheter, and the wall(s) of the expandable funnel may be formed of a mesh cover. In such variations the device may be adapted to prevent the tines from poking through the distal end of the funnel and catching on the tractor, jamming the apparatus. This may be achieved by, for example, forming a capture tab on the tine at the distal end of each tine to hold a connecting filament (e.g., suture) so that it extends between the tines but is unable from being displaced distally by the capture tab. In some variations the capture tab may be cut as a tab in the side(s) of the tine and bent up to lock the filament in position.

Thus, in general, described herein are apparatuses that include an expandable funnel on the end of an inversion support catheter over which a flexible tube (e.g., tractor tube or simply tractor) rolls to invert. The flexible tube may be a knitted, or woven material. The flexible tube may generally have a first end coupled at a distal end region of a puller (which may be an elongate wire, tube, cannula, etc.), and the flexible tube may be arranged to invert over a distal end of the inversion support catheter at the funnel so that an external portion of the flexible tube extends proximally over the inversion support catheter as the internal (inverted) portion of the flexible tube is drawn into the funnel, compressing and removing fluid from the clot that is held by (e.g., grabbed by) the flexible tube, and pulling the inverted flexible tube into the inversion support catheter until the entire clot is captured, compressed and drawn into the outer catheter. The configuration of the apparatuses described herein are particularly well suited to grabbing and removing clot, and particularly large diameter clots, by using an inversion support catheter than includes an expandable funnel at the distal end. The collapsible/expandable funnel may be configured to operate with the compressive forces applied by pulling the flexible tube into the inversion support catheter so that it inverts into the inversion support catheter, capturing the clot. The collapsible/expandable funnel may be configured to assume a fully expanded, locked (e.g., "jammed") configuration when the flexible tube applies a laterally compressive force on the distal end face of the funnel. Further, the funnel may include openings (described herein as having a porous structure) through which fluid squeezed out of the clot may exit laterally out of the funnel as the clot is moved into the narrower-diameter lumen of the inversion support catheter and compresses the clot. For example, the openings through the collapsible/expandable funnel (which may be referred to herein as simply an expandable funnel) that permit fluid to laterally leave the funnel walls as the clot is compressed may also prevent clogging or jamming of the clot.

Any of these apparatuses may be configured to help break up the captured material within the funnel. According to the claimed invention, described herein are apparatuses for removing material from a body lumen, the apparatus comprising: an inversion support comprising a catheter having a catheter lumen and an elongate and flexible catheter body, and an expandable funnel disposed at a distal end of the catheter body and extending in a distal to proximal axis, wherein a distal end of the funnel defines a distal end opening in communication with an interior of the funnel and the catheter lumen, respectively, further wherein, in an open configuration, the interior of the funnel has a wall angle of less than 14 degrees relative to the proximal to distal axis for a majority of a length of the funnel and includes a first region in which the wall angle is between 14 and 50 degrees with respect to the distal to proximal axis; and a tractor comprising a flexible tube that extends distally in an un-inverted configuration along an outer surface of the catheter, inverts over the distal end opening and extends proximally within the catheter lumen in an inverted configuration, wherein the flexible tube is configured to invert by rolling over the distal end opening when a first end of the tractor is pulled proximally within the catheter lumen.

As mentioned, the funnel may comprise a mesh forming the interior of the funnel and an exterior of the funnel. The funnel may include a second region within the interior of the funnel in which the wall angle is between 14 and 50 degrees with respect to the distal to proximal axis, wherein the second region is separated from the first region by an intermediate region in which the wall angle is less than 14 degrees relative to the distal to proximal axis.

For example, an apparatus for removing material from a body lumen (e.g., removing a clot from a blood vessel), may include: an inversion support comprising a catheter having a catheter lumen and an elongate and flexible catheter body, and an expandable funnel disposed at a distal end of the catheter body and extending in a distal to proximal axis, wherein a distal end of the funnel defines a distal end opening in communication with an interior of the funnel and the catheter lumen, respectively, further wherein, in an open configuration, the interior of the funnel comprises one or more constricted regions wherein the interior of the funnel constricts from the proximal to distal direction; and a tractor comprising a flexible tube that extends distally in an un-inverted configuration along an outer surface of the catheter, inverts over the distal end opening and extends proximally within the catheter lumen in an inverted configuration, wherein the flexible tube is configured to invert by rolling over the distal end opening when a first end of the tractor is pulled proximally within the catheter lumen.

Any of these apparatuses may include a funnel with one or more constrictions within the inner lumen of the funnel. For example, at least one portion of the interior of the funnel having the wall angle of less than 14 degrees relative to the proximal to distal axis has a negative wall angle relative to the distal to proximal axis, such that the interior of the funnel constricts from the proximal to distal direction. In general, the wall angle of the interior of the funnel is measured between the proximal to distal axis and the wall of the interior of the funnel from the distal-facing direction.

In general, the exterior of the funnel may have a different wall profile than the wall profile of the interior of the funnel. For example, in some variations, the entire exterior of the funnel has a wall angle that is less than 14 degrees with respect to the proximal to distal axis (in the distal facing direction).

Any of the funnels described herein may include openings allowing passage of fluid from the material as it is compressed when being drawn into the funnel. For example, at least the base region of the funnel adjacent to the distal end of the catheter body may include openings configured to allow fluid to pass therethrough. In some variations the funnel comprises a circumferential porous region at a base of the funnel adjacent to the distal end of the catheter body, the porous region configured to allow fluid to pass therethrough.

The funnel may have a collapsed configuration having a maximum outer diameter of less than 0.3x an outer diameter of the catheter body proximal of the funnel. The funnel may have an open (e.g., expanded) configuration in which the funnel has a minimum outer diameter of greater than 1.5x an outer diameter of the catheter body proximal of the funnel.

The funnel may be any appropriate size for insertion into a body lumen and for opening within the lumen (e.g., blood vessel). For example, the funnel may have an outer diameter of between 2 and 26 mm in the open configuration.

As mentioned above in any of these variations, the flexible tube may comprise a knitted tube.

The funnel may be configured to open into the open configuration from a collapsed (e.g., unexpanded) configuration when the flexible tube is pulled proximally into the catheter lumen and exerts an axial compressive force on the distal end of the funnel.

In any of these apparatuses and methods, the funnel may be held in a jammed state (in the open configuration) when the flexible tube is pulled proximally into the catheter lumen. Thus, the funnel may be opened and locked (jammed) in a high column strength jammed state by pulling the tractor into the distal end opening of the funnel. The funnel may have a greater column strength in the jammed state as compared to the un-opened funnel and/or the funnel in the un-jammed state. For example, the funnel may be configured to withstand greater than 5 Newtons of compressive force without collapsing in the jammed state (e.g., 7 Newtons or greater, 8 Newtons or greater, than 10 Newtons or greater, 11 Newtons or greater, 12 Newtons or greater, 13 Newtons or greater, 14 Newtons or greater, 15 Newtons or greater, 18 Newtons or greater, 20 Newtons or greater, etc.).

In any of these apparatuses, the funnel may include a plurality of longitudinal tines that are continuous with the catheter body proximal of the funnel. The funnel may additionally or alternatively include a mesh inverted over itself forming an inner wall of the funnel and an outer wall of the funnel, and wherein the plurality of longitudinal tines are positioned between the inner wall of the funnel and the outer wall of the funnel. In some variations the apparatus (e.g., the funnel) may include a filament connecting the ends of the tines; the filament may be locked in position at the distal end of the tines, in order to prevent the tines from projecting through the covering mesh (e.g., a knitted or woven mesh) during operation of the apparatus, which may otherwise snag the tractor as it is pulled into the distal end opening.

As mentioned, any of these apparatuses may include a puller disposed within the catheter lumen, wherein the first end of the tractor is coupled to the puller.

Also described herein are methods of removing a material from a body lumen, the method comprising: advancing an inverting tractor apparatus through the body lumen until a distal end portion of the apparatus is located proximate to the material, wherein the inverting tractor apparatus comprises an inversion support comprising a catheter having a catheter body and an internal catheter lumen, and an expandable funnel disposed at a distal end of the catheter body and extending in a distal to proximal axis, wherein a distal end of the funnel defines a distal end opening in communication with an interior of the funnel and the catheter lumen, respectively, the inverting tractor apparatus further including a tractor comprising a flexible tube that extends distally in an un-inverted configuration along an outer surface of the catheter; pulling a first end of the tractor proximally within the catheter lumen so that the tractor inverts over the distal end opening, further wherein pulling the first end of the tractor proximally exerts an axial compressive force on the distal end of the funnel and opens the funnel into an open state from a collapsed state; and pulling the material into a first distal region of the funnel having a wall angle of less than 14 degrees relative to a proximal to distal axis of the funnel, then pulling the material into second, more proximal, region of the funnel having a wall angle that is between 14 and 50 degrees with respect to the distal to proximal axis, whereby the material is compressed and extruded into the catheter lumen. Any of these methods may be methods of removing a blood clot (thrombus) from a blood vessel.

For example, a method of removing a material from a body lumen may include: advancing an inverting tractor apparatus through the body lumen until a distal end portion of the apparatus is located proximate to the material, wherein the inverting tractor apparatus comprises an inversion support comprising a catheter having a catheter body and an internal catheter lumen, and an expandable funnel disposed at a distal end of the catheter body and extending in a distal to proximal axis, wherein a distal end of the funnel defines a distal end opening in communication with an interior of the funnel and the catheter lumen, respectively, the inverting tractor apparatus further including a tractor comprising a flexible tube that extends distally in an un-inverted configuration along an outer surface of the catheter; pulling a first end of the tractor proximally within the catheter lumen so that the tractor inverts over the distal end opening, further wherein pulling the first end of the tractor proximally exerts an axial compressive force on the distal end of the funnel and opens the funnel into an open state from a collapsed state; and pulling the material into a first, distal, region of the interior of the funnel having, then pulling the material into second, more proximal, region of the interior of the funnel, wherein the first region and the second region are separated by a constriction narrowing an inner diameter of the interior of the funnel, whereby the material is compressed and extruded into the catheter lumen.

In any of these methods and apparatuses, the elongate inversion support portion of the apparatus described herein may be or may include (particularly at its distal end) any appropriate catheter, e.g., a flexible tube that can be inserted into a body vessel (e.g., blood vessel) into which the more flexible tractor portion can be withdrawn by pulling against the elongate inversion support. The elongate inversion support may, in some variations, also be referred to as outer catheters (e.g., when the puller for the tractor is referred to as an inner catheter) and/or inversion catheters and/or support catheter, as it may support the inversion of the tractor. The elongate inversion support, including a catheter forming the elongate inversion support, may include a braided or woven portion, a spiral or coiled portion, etc. (e.g., having a braided shaft), may have a single layer or multiple layers, and may be formed of biocompatible materials, including polymers, metals, etc. (e.g., PTFE). Examples of vascular catheters that may form the elongate inversion support include micro catheters.

As mentioned, the apparatuses described herein may be mechanical thrombectomy apparatuses and may include a tractor region and/or elongate inversion support that are configured to prevent locking onto the outside of the inversion support, while still able to efficiently "grab" a clot from within a body lumen. For example, described herein are mechanical thrombectomy apparatuses that may be configured to grab or grasp and/or macerate a clot as it is mechanically drawn into the apparatus for removal. Although suction may be used in addition to the mechanical grabbing of the clot, in some variations suction is not used.

The tractor regions described herein may include projections that extend from the tractor region, particularly or exclusively as it bends around during inverting (e.g., at the distal end of the device). These projections may remain flat or non-extending when the tractor is held in parallel with the elongate inversion support. Alternatively, the projections may extend at all times. In general, the tractor may be formed of woven material, knitted material, or laser-cut sheet of material. The knitted and/or woven materials may be fibrous materials (including natural fibers, synthetics fibers, etc.), polymeric materials, or the like. For example, the material (e.g., strands) forming the woven or knitted material may be one or more of: monofilament polymer, multifilament polymer, NiTi filament, NiTi tube with radiopaque metallic center, Cobalt chromium alloy filament, Cobalt chromium alloy tube with radiopaque metallic center, Nylon, Polyester, Polyethylene terephthalate, and Polypropylene. The sheets of material (e.g. a solid sheet of material) formed into the tractor region may be one or more of: polymeric material (e.g., PTFE), silicone materials, polyurethanes, shape memory alloys, stainless steels, etc. The sheets may be extruded, glued, or the like. The sheets may be cut to form pores and/or projections. For example, the sheets may include one or more laser-cut projections. Any of these apparatuses may be coated with a hydrophilic and/or hydrophobic coating, and/or may include pores. The tractor may have a porosity of greater than >60% (greater than 70%, greater than 75%, greater than 80%, greater than 85%, etc., between 60-95, 65-95, 70-95%, etc.).

For example, described herein are clot-grabbing mechanical thrombectomy apparatuses that include a tractor region. The tractor region may include a plurality of clot-grabbing projections extending from one face of the tractor. In some variations, the clot-grabbing projections may be configured so that they move to extend (e.g., out of the plane of the tractor) when the tractor region bends around, e.g., around the distal end of the catheter of the elongate inversion support, to invert.

The tractor may be configured (e.g., by heat setting, shape setting, etc.) to maintain the portion of the tractor within the catheter, in the inverted configuration, so that it is close to the inner diameter of the catheter; e.g., the inner diameter of the portion of the tractor within the catheter may be greater than 50 % of the inner diameter of the catheter, greater than 55% of the inner diameter of the catheter, greater than 60% of the inner diameter of the catheter, greater than 65% of the inner diameter of the catheter, greater than 70% of the inner diameter of the catheter, greater than 75% of the inner diameter of the catheter, etc.

Further, in any of the apparatuses described herein, the tractor in the un-inverted configuration maintains an outer diameter that is slightly larger than the outer diameter of the inversion support catheter. The tractor may be biased to maintain an outer diameter that is within about 2 mm, within about 1.5 mm, within about 1 mm, within about 0.8 mm, within about 0.7 mm, within about 0.6 mm, within about 0.5 mm, etc. of the OD of the inversion support catheter.

Any of the apparatuses described herein may include a tractor having one or more coatings from the group of: a lubricious coating, a metal coating, a heparin coating, an adhesive coating, and a drug coating.

## BRIEF DESCRIPTION OF THE DRAWINGS

A better understanding of the features and advantages of the disclosed apparatuses and methods of using same will be obtained by reference to the following detailed description when read in conjunction with reviewing the accompanying drawings, in which:

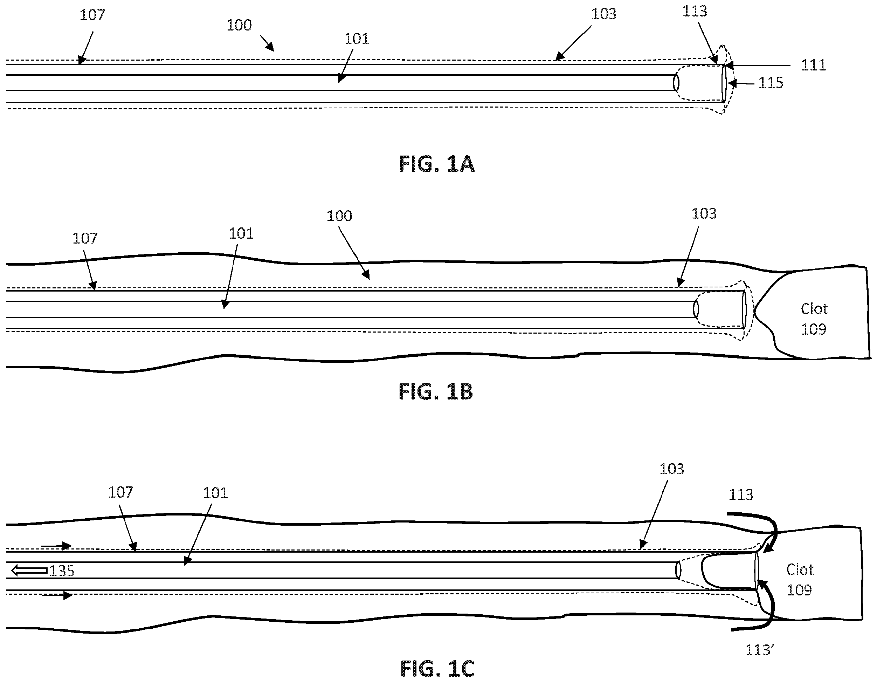

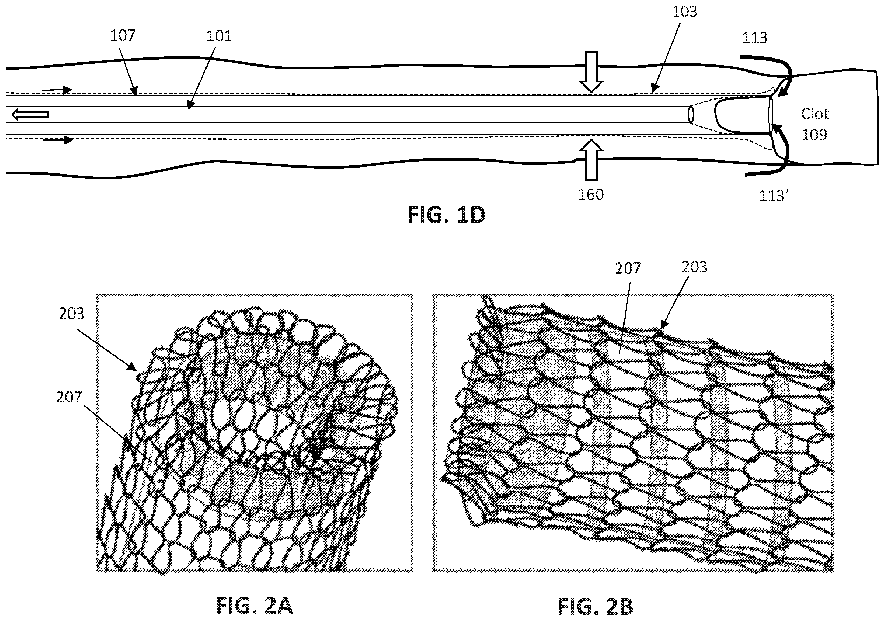

FIGS. 1A-1D illustrate an example of an apparatus for mechanically removing an object such as a clot form a body region. FIG. 1A shows an example of an apparatus including an elongate inversion support including a catheter. For example, at least the distal end of the elongate inversion support may be configured as a catheter. The apparatus also includes a flexible tube configured as a tractor that may be pulled into the lumen of the inversion support. FIG. 1B shows the apparatus of FIG. 1A within a lumen (e.g., blood vessel) and positioned adjacent to a material to be removed (e.g., a clot). FIG. 1C shows the apparatus of FIG. 1A being actuated to remove the material. FIG. 1D shows one potential failure mode of an apparatus such as that shown in FIGS. 1A-1C, in which the tractor, which may be configured to reside (in a relaxed configuration) near the outer surface of the inversion support, may choke down onto the inversion support, preventing operation of the apparatus.

FIGS. 2A-2B illustrate end perspective and side views, respectively, of an apparatus such as the one shown in FIGS. 1A-1D with a tractor formed of a knitted material, as shown.

FIG. 3A illustrates one example of a tortious anatomical region, shown as the Ilioa-Caval arch through which an apparatus such as described herein (e.g., an inverting tube apparatus) may be navigated.

FIG. 3B shows one example of a prototype apparatus including a cuff encloses a portion of the distal end region of the tractor on the outside of the inversion support.

FIG. 4A shows an enlarged view of one example of a knitted tractor region, showing generally teardrop-shaped loops. FIGS. 4B and 4C show examples of knitted tractors having 22 needle (e.g., 22 loops per turn) and 34 needle (e.g., 34 loops per turn), respectively.

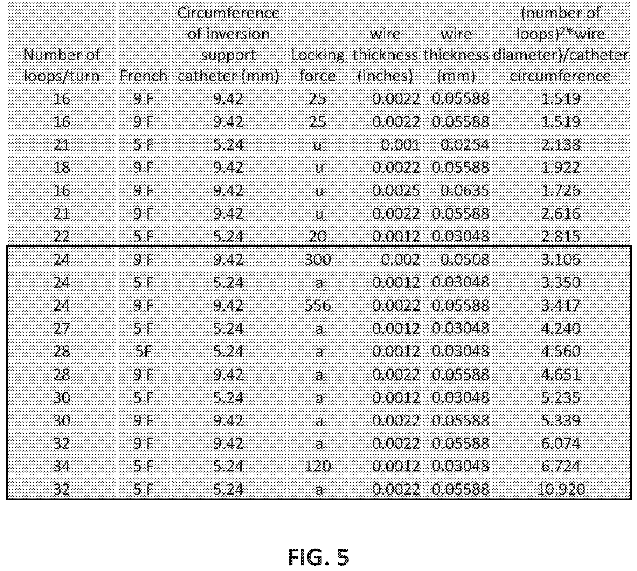

FIG. 5 shows table 1, annotating examples of tractors of different parameters examined for suitability based on the force applied before the device locks onto the outer diameter of the inversion support catheter. The different parameters illustrated in this table include the number of loops per turn, the size of the catheter onto which the tractor is to be used, the thickness of the filament ("wire") used to form the knitted tractor, and the locking force at which the tractor locked onto the inversion support catheter. In these experiments a longer length of tractor formed as described in the chart (e.g., from a knitted filament having the indicated thickness and number of loops) was examined by pulling the tractor on the inversion support catheter of the dimensions shown until the tractor locked onto the inversion support catheter.

FIG. 6 is an example of an inverting tube apparatus similar the one shown in in FIG. 1A in a large vessel, having clot with a diameter that is greater than 2x the inner diameter of the inversion support catheter. Although the clot may be ingested and removed by the inverting tube apparatus having a narrower-diameter inversion support catheter, the efficiency of the inversion may be low, particularly where the clot is made of a hard (e.g. ,partially calcified) material.

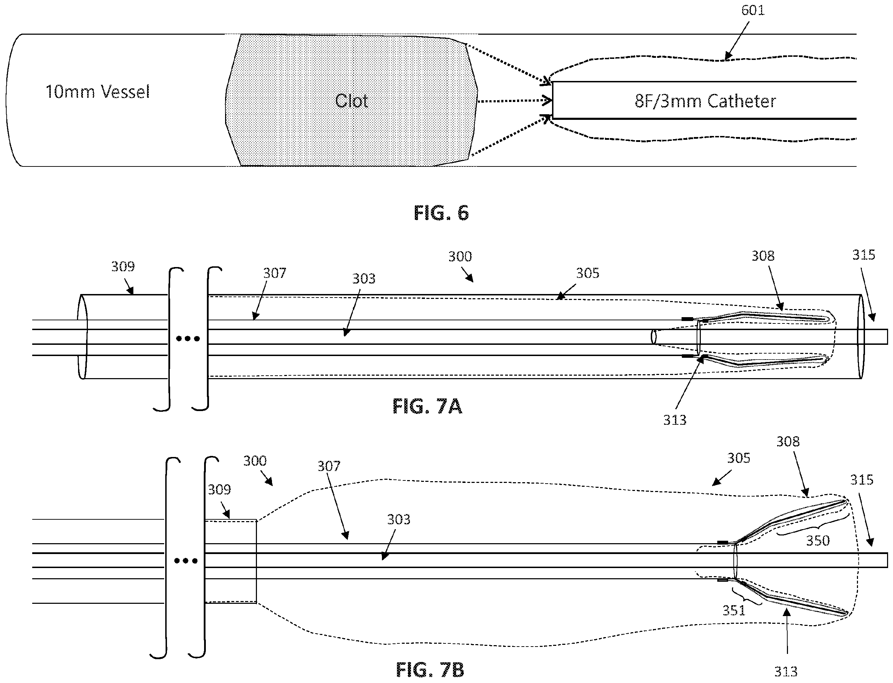

FIGS. 7A-7B shows an example of an inverting tube apparatus as descried herein that is adapted to dehydrate (e.g. remove liquid) from the material, e.g., clot, as it is ingested, increasing the efficiency of the inversion, in which the funnel includes internal regions of different wall angles that may assist in breaking up and compressing harder clots. In FIG. 7A the inverting tube apparatus is shown in an undeployed stated, within an intermediate (e.g., delivery) catheter; the inversion support catheter includes an expandable funnel at the distal end over which a flexible tube inverts. The flexible tube is attached at a distal end region of the puller so that the puller may extend distally. In FIG. 7B the inverting tube apparatus of FIG. 7A is shown in a deployed configuration with at least the distal end extending from the intermediate catheter; the expandable funnel at the distal end of the inversion support catheter is in an open configuration.

FIGS. 8A-8B illustrate another example of an inverting tube apparatus including an expandable funnel having different internal wall angles, in which the flexible tube (e.g., knitted tube) is attached to distal end of a puller. FIG. 8B shows the inverting tube apparatus of FIG. 8A in a deployed configuration with the intermediate (e.g., delivery) catheter withdrawn proximally so that the expandable funnel at the distal end of the inversion support catheter can expand, and the flexible tube can expand.

FIG. 8C is an image of a prototype of an inverting tube apparatus similar to that shown in FIGS. 8A-8C.

FIGS. 9A-9B show an example of the distal end of an inverting tube apparatus including a funnel at the distal end of the inversion support catheter, over which the knitted flexible tube rolls and inverts. FIG. 9A is a top perspective view and FIG. 9B is a side view.

FIGS. 10A-10C illustrate examples of inversion support catheters with expandable funnels at their distal ends.

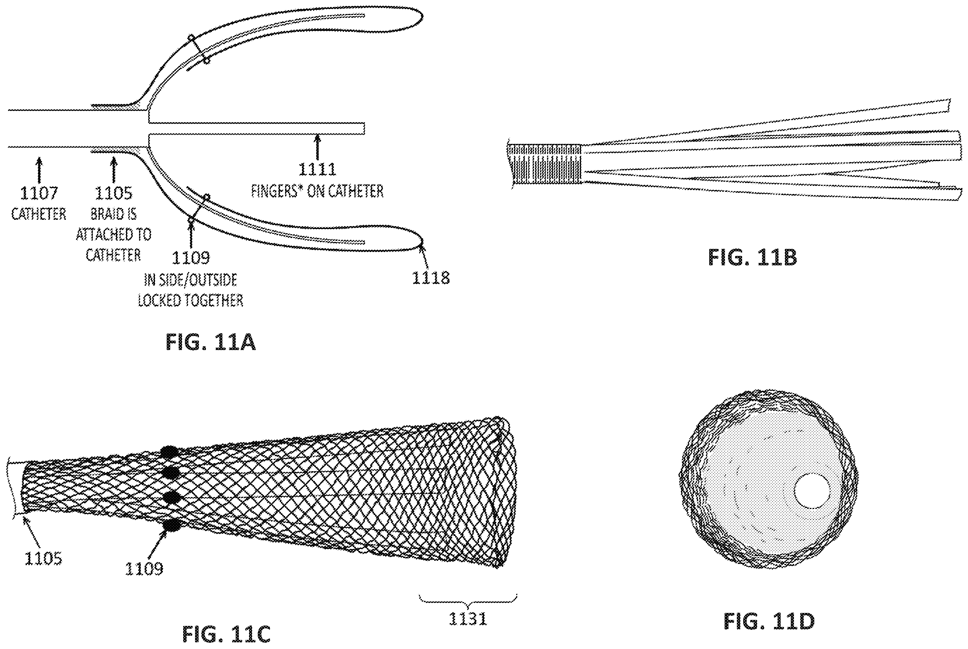

FIG. 11A-11D illustrates an example of an inversion support catheters with expandable funnels. FIG. 11A is a schematic illustration of an inversion support catheter including a funnel including a support frame that is formed from the distal end region of the elongate inversion support catheter. FIG. 11B shows an example of an inversion support catheter cut to form the supports shown schematically in FIG. 11A. FIG. 11C is an example of a distal funnel of an inversion support catheter formed by a braided material attached to a frame such as the frame shown in FIG. 11B. FIG. 11D is an end view of the funnel shown in FIG. 11C.

FIG. 12 is an example of a funnel of an inversion support catheter that is configured to have an axial compressive strength that is sufficient to resist collapsing when greater than 500g (e.g., greater than 1 kg, greater than 1.2 kg, greater than 1.5 kg, etc.) of axial compressive force is applied. In this example, the axial compressive force, which may be applied by the flexible tube being drawn proximally (e.g., pulled) to roll over the distal end of the funnel, may also open the funnel to deploy it.

FIG. 13 is an example of an expandable funnel of an inversion support catheter in a relaxed state.

FIG. 14 is another example of an expandable funnel of an inversion support catheter.

FIG. 15A is an example of a distal end of an inversion support including an expandable distal funnel, shown in the expanded state. In this example the funnel includes a small (e.g., less than 14 degree) wall angle on the outer and inner walls.

FIG. 15B shows another example of a distal end of an inversion support including an expandable distal funnel, shown in the expanded state.

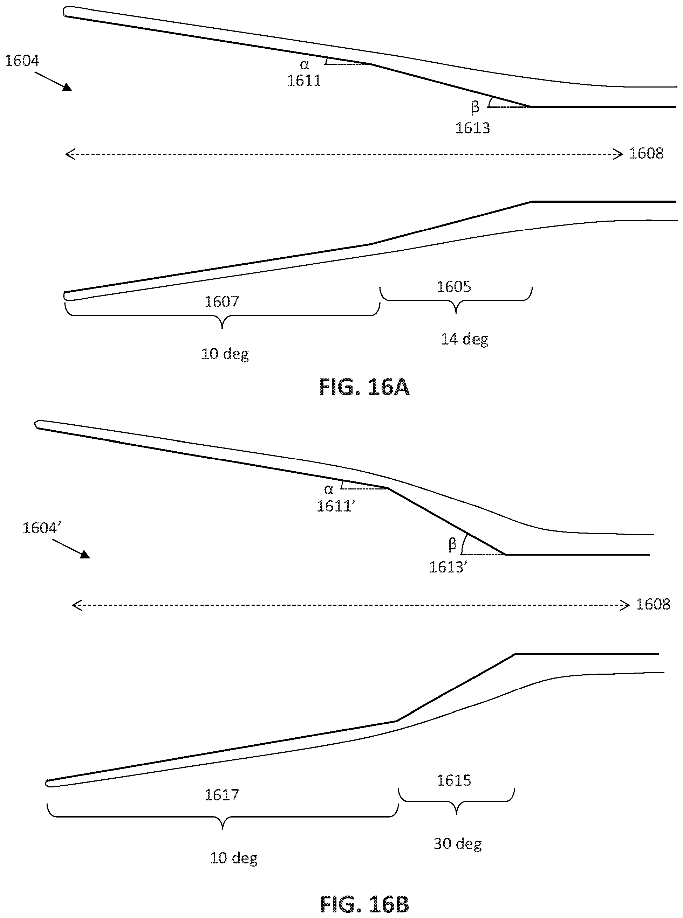

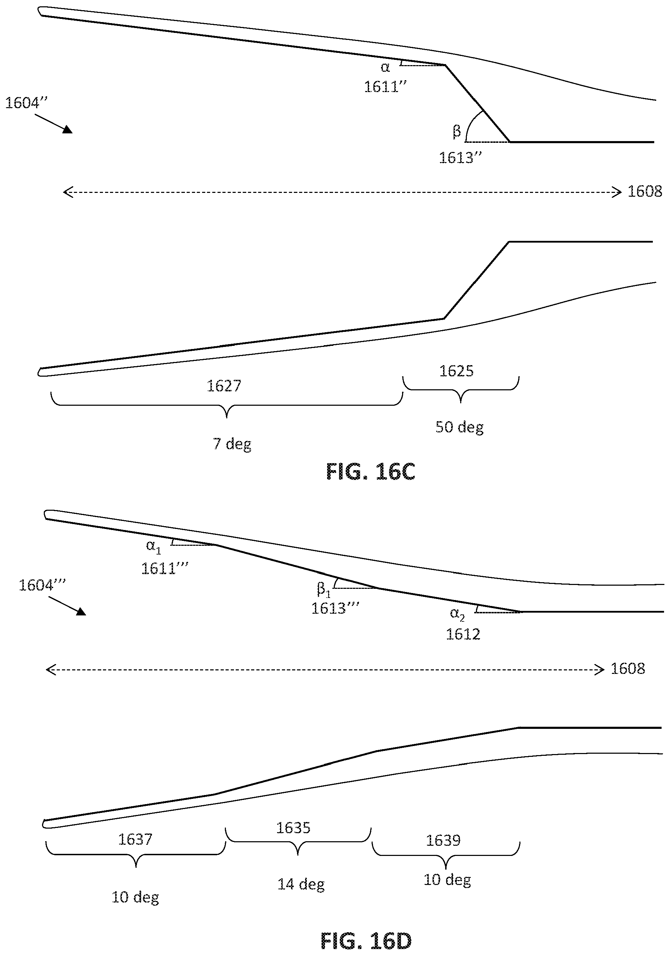

FIGS. 16A-16D illustrate example of schematic cross-sections through a distal end of an inversion support including an expandable distal funnel, showing lumen regions of different wall angle. In FIG. 16A the inner lumen includes a first region of wall angle approximately 10 degrees (maximum) and a second region of wall angle approximately 14 degrees (maximum). FIG. 16B shows an inner lumen includes a first region of wall angle approximately 10 degrees (maximum) and a second region of wall angle approximately 30 degrees (maximum). FIG. 16C shows an inner lumen includes a first region of wall angle approximately 7 degrees (maximum) and a second region of wall angle approximately 50 degrees (maximum). FIG. 16D shows an example of an inner lumen of the funnel having three different wall angles, including a first region having a wall angle of 10 degrees or less, a second region having a wall angle of 14 degrees (e.g., between 14-50 degrees) and a third region having a wall angle of 10 degrees or less.

FIG. 17 shows another example of a prototype of an inversion support including a distal end that is configured as a funnel having one or more constricted regions within the inner lumen that may help in capturing hard materials.

FIGS. 18A-18B illustrate examples of cross-sections through a funnel of an inversion support similar to that shown in FIG. 17, having two constricted regions.

FIG. 19 is an example of a prototype funnel in which the tines supporting the funnel have protruded through the distal end covering forming the outer surface of the funnel.

FIGS. 20A-20B illustrate examples of a set of tines forming a funnel of an inversion support including a bridging element, shown in this example as a suture (restraining filament),

FIGS. 21A-21D illustrate the formation of an integral proximal restraint for a restraining filament formed from the tines of the funnel.

FIGS. 22A-22B show side and end perspective views, respectively, of a tine including a proximal restraint formed integrally from the tine, as described herein. This tine may be part of a funnel.

## DETAILED DESCRIPTION

In general, described herein are apparatuses having an inverting tractor that is configured to roll into an inversion support catheter and capture material from within a vessel. In particular, described herein are methods and apparatuses that are configured to capture material with an inverting tractor in which the tractor is a knitted tractor that is configured to prevent lock down of the knitted tractor on the outside of the inversion support (e.g., inversion support catheter). Also described herein are apparatuses in which the inversion support catheter includes an expandable funnel at the distal end having an interior profile that is adapted to capture and break apart hard material captured by the tractor so that it may be pulled into the inversion support catheter for removal.

For example, these apparatuses may include knitted tractors that are configured so that even longer tractors (e.g., greater than 20 cm) that are held close the outside of the inversion support (e.g., within 2 mm of the outer diameter of inversion support catheter) do not lock down onto the outside of the inversion support when pulled over the distal end opening and into the inversion support catheter. The tractor may have a knitted tube that is configured to stretch less than 3 percent when pulled in tension with a force of 2 Newtons. For example, in some variations, the tractor may include a woven arrangement of a number of N loops per turn formed of a filament having a filament diameter, wherein the ratio of a square of N loops per turn multiplied by the filament diameter, per the circumference of the catheter that is greater than 2.9 (e.g., greater than 3.0, etc.). The ranges of dimensions and number of loops per turn of the knit may be selected as described herein to prevent stretch for longer tractors while maintaining flexibility and the ability of the tractor to capture material from the vessel, without increasing the force necessary to pull the tractor into the inversion support catheter.

The apparatuses described herein may generally be configured to include a knit tractor that is configured to prevent jamming, including locking down on to the inversion support catheter. The apparatuses described herein may generally include an elongate inversion support that supports an annulus over which the tractor inverts at the distal end. The tractor may comprise a flexible (e.g., knitted) tube that doubles back over (e.g., inverts) over the distal end of the elongate inverting support (e.g., a catheter) so that it extends into the annular opening of the elongate inversion support. In some variations an inner puller may be coupled to one end of the tractor that the tractor can be pulled proximally to invert over the distal end opening (annulus) at the distal end of the elongate inverting support to roll and capture a material within the vessel. The apparatus may include a guidewire lumen extending through the elongate inverting support, and/or tractor puller that is configured to pass a guidewire.

The methods and apparatuses described herein may prevent locking down of the knitted tube onto the outside of the inversion support catheter. For example, described herein are apparatuses and methods for removing a material from a vessel including a tractor portion that is configured so that, even when the tractor tube is held in close proximity to the outer surface of the inversion support catheter (e.g., within 1.5 mm, within 1 mm, within 0.8 mm, within 0.5 mm, within 0.4 mm, within 0.2 mm, etc., in some variations on average), the knitted tube does not choke down and lock onto the outer surface of the inversion support catheter when pulled into the inversion support catheter, even at longer lengths. For example, the tractor may be configured so that it does not stretch (and therefore constrict) more than a predefined percentage when pulled in tension. The tractor may be configured so that it does not stretch more than 3 percent when pulled in tension with a force of 2 Newtons. In general, the tractor may be configured so as not to stretch while still remaining sufficiently flexible so as to roll over the distal end of the catheter easily, capture clot, and not jam within the distal end opening of the inversion support catheter.

Thus, described herein are particular configurations of the knitted tractor, such as the dimensions of the knitted filament forming the tractor (e.g., filament width), the number of loops of the knitted tractor per turn, and the size (e.g., circumference) of the inversion support catheter over which the tractor tube is to be pulled. The material forming the knitted tractor (e.g., stainless steel, Nitinol, etc.) may also contribute to the device performance, as may the size (e.g., the length) of the loops may also contribute. Without being bound by a particular theory of operation, described herein are methods and apparatuses in which the relationship between the number of loops per turn, the circumference of the inversion support catheter and the thickness (e.g., cross-sectional thickness) of the filament forming the loops of the knitted tractor may define a range of values that have been found empirically, as described herein, to provide a knitted tractors that resist locking down on to the inversion support catheter. Surprisingly, outside of the specified range, knitted tubes may otherwise lock onto the inversion support catheter.

Any of the apparatuses described herein may also include a coating (e.g., hydrophilic, lubricious coating, etc.) or the like to enhance the sliding and inverting of the tractor over the distal end. Further, any of these apparatuses may include one or more projections that are configured to enhance grabbing and/or maceration of a clot. Grabbing of a clot may be particularly, but not exclusively, helpful when the tractor is lubricious. Although lubricious tractors may resist jamming and require less force to operate, e.g., inverting over the distal end of the catheter, it may be more difficult to initially grab or grasp clot when the tractor is more lubricious. It may also be particularly helpful to include projections that are retracted along the length of the tractor adjacent to the outer diameter of the elongate inverting support (e.g., catheter), for example, when positioning the apparatus within a vessel, but extend the projections outward from the tractor when rolling and inverting to grab a clot.

In general, an apparatus for removing a material from a vessel may be a system, assembly or device including an elongate inversion support having a distal end and a distal annulus, and a flexible tractor assembly at least partially inverted and configured to roll and invert over the distal annulus of the elongate inverting support.

In many of the examples described herein, the elongate inversion support is a catheter (or a portion of a catheter at the distal end) and the annulus is formed by the distal end opening of the catheter; the tractor extends within the catheter and doubles back over the distal end of the catheter to extend over the outer diameter of the catheter at the distal end of the catheter, although it may extend proximal for any appropriate distance (including greater than 20 cm, e.g., greater than 30 cm, between 20-200 cm, greater than 40 cm, 50 cm, 60 cm, 70 cm, 80 cm, 90 cm, 100 cm, 110 cm, 120 cm, 130 cm, 140 cm, 150 cm, 160 cm, 170 cm, 180 cm, 200 cm, etc.). The end of the tractor within the catheter may be coupled to a puller (e.g., at a proximate puller region connected to the distal or inner end of the tractor). The tubular tractor and puller may include an elongate lumen that is configured to allow passage of a guidewire. The tubular tractor may also be configured to slide along the long axis within the catheter lumen and invert over the distal end opening of the catheter when the proximal end region is pulled proximally. The tractor may be referred to herein as a tractor assembly, tractor portion, tractor tube, or simply a tractor, and is typically positioned and longitudinally slideable within the catheter, and arranged so a portion of the tractor (sometimes referred to as the "distal tractor region" or "distal-facing" tractor region) doubles back over itself.

For example, FIG. 1A shows one variation of an apparatus 100, including a catheter of an elongate inversion support. In this example, the elongate inversion support includes a catheter 107 having a distal end region 113 that includes a distal end opening 115. The distal end region may have an increasing softness (measured by durometer, e.g., shore durometer) except that the very distal-most end region (distal end 115, including the distal end opening) may be substantially less soft than the region immediately proximate to it. Thus, although the distal tip region of the catheter (e.g., the distal most x linear dimensions, where x is 10 cm, 7 cm, 5 cm, 4 cm, 3 cm, 2 cm, 1 cm, 9 mm, 8 mm, 7 mm, 6 mm, 5 mm, 4 mm, 3 mm) has an increasing softness/decreasing harness extending from the proximal to distal ends, the very distal end region (e.g., measured as distal most z linear dimensions, where z is 1 cm, 9 mm, 8 mm, 7 mm, 6 mm, 5 mm, 4 mm, 3 mm, 2 mm, 1 mm, 0.8 mm, 0.5 mm, 0.3 mm, 0.2 mm, etc., and z is always at least three times less than x) has a hardness that is greater than the hardness of the region immediately proximal to it, and may be as hard or harder than the proximal-most region of the distal tip region.

In FIG. 1A, the elongate inversion support is an elongate hollow catheter having a column strength that is sufficient to prevent buckling when the catheter is pulled over the distal annulus (distal end opening). Thus, the elongate inversion support may be configured so that it does not collapse (e.g., buckle) when 500 g (1 g of force = 0.00981 N) or less of compressive force is applied (e.g., at least about 700 g, 600 g, 500 g, 400 g, 300 g, etc. of compressive force) for neurovascular applications. For peripheral vascular applications the elongate inversion support may be selected or configured to withstand at least 1500 g of compressive force (e.g., at least about 2000 g, 1900 g, 1800 g, 1700 g, 1600 g, 1500 g, 1400 g, etc. of compressive force). In general, any of the apparatuses described herein may include an elongate inversion support that is not a full-length catheter, but may include a portion of a catheter, typically at the distal end, connected to a rod, wire, hypotube, or the like or may be skived. In some variations the distal end 115 of the elongate inversion support is adapted so that the tractor 103 may slide or roll and invert over the distal end of the catheter without being caught (binding, jamming) or without substantial friction.

The apparatus shown in FIG. 1A also includes a puller 101 to the tractor 103 at or near the distal end or the puller (in some variations the tractor may be attached at a slightly proximal region of the puller, so that the puller distal end extends forward ahead of the puller when extended distally from the inversion support catheter. The tractor may therefore roll over the distal end opening 111 of the inversion support catheter. In this example the tractor 103 is configured to ride over the outer diameter of the inversion support so that it is within, e.g., about 1 mm or less (e.g., 0.5 mm or less) of the outer diameter of the inversion support catheter.

FIG. 1B shows the apparatus of FIG. 1A within a vessel of a body (e.g., a blood vessel) in which a clot 109 is located. The proximal end of the apparatus may be positioned adjacent to the clot. In some variations a delivery catheter (not shown) may be positioned within the vessel and the inversion support catheter and tractor (and in some variations puller) may be driven through the delivery catheter, e.g., over a guidewire, so that it is adjacent to the clot. FIG. 1C shows an example of a flexible tractor 103 coupled to a puller 101. In this example the tractor 103 is integrated with the puller 101, forming an assembly. In FIG. 1C, the tractor is a tube of material (e.g., knitted) that is flexible and elongate (longer than 20 cm). The tractor has a relaxed inner diameter that is slightly greater than the outer diameter of the catheter of the elongate inversion support into which the tractor will be pulled. The flexible and tubular tractor 103 may be sufficiently soft and flexible (e.g., having a low collapse strength) so as to easily roll and fold over the distal aperture of the elongate inversion support. The puller 101 may be, e.g., a hypotube. In some variations a puller is not necessary, but the internal end of the tractor may extend proximally and be directly pulled.

In FIG. 1C, the tractor 103 is configured, e.g., by shape-setting (heat setting, etc.), to expand in the relaxed inverted configuration (when inverted within the inversion support catheter) to a radial diameter that is at least 60% of the inner diameter of the inversion support catheter (e.g., at least 65%, at least 70%, at least 75%, at least 80%, at least 85%, at least 90%, at least 95%, at least 100%, etc., e.g., between 0.7 and 4 times, between 0.8 and 4 times, between 0.7 and 2 times, etc.) when unconstrained. This may prevent the inverted tractor from collapsing down onto itself in the inverted configuration, even when pulled proximally into the catheter. This may also for the outward-flared shape as it inverts into the distal end opening of the catheter.

FIG. 1C shows the operation of the apparatus of FIGS. 1A-1B to draw clot material into the inversion support catheter. In this example the puller 101 is pulled proximally (arrow 135), while the inversion support catheter is either held steady or advanced distally; this pulls the knitted tractor 103 over the distal end opening of the inversion support catheter so that it rolls 113 and inverts into the inversion support catheter, drawing clot material with it.

In general, it has been found that longer tractors, and in particular longer knitted tractors, such as knitted tractors that are longer than about 20 cm (e.g., about 25 cm or longer, about 30 cm or longer, about 35 cm or longer, about 40 cm or longer, about 45 cm or longer, about 50 cm or longer, about 60 cm or longer, about 70 cm or longer, about 80 cm or longer, about 100 cm or longer, about 110 cm or longer, about 140 cm or longer, about 150 cm or longer, etc.) may lock up because of friction between the outside of the inversion support catheter and the tractor, as shown in FIG. 1D. In FIG. 1D, the arrows 160 show the tractor choking down onto the outside of the inversion support catheter 107. This may be particularly problematic where the tractor in the relaxed configuration has an inner diameter that is within a few mm (e.g., within about 2 mm, within about 1.5 mm, within about 1 mm, within about 0.8 mm, etc.) of the outer diameter of the inversion support catheter. FIGS. 2A-2B illustrate one example of a knitted tractor 203 that is extending over the outside of an inversion support catheter 207.

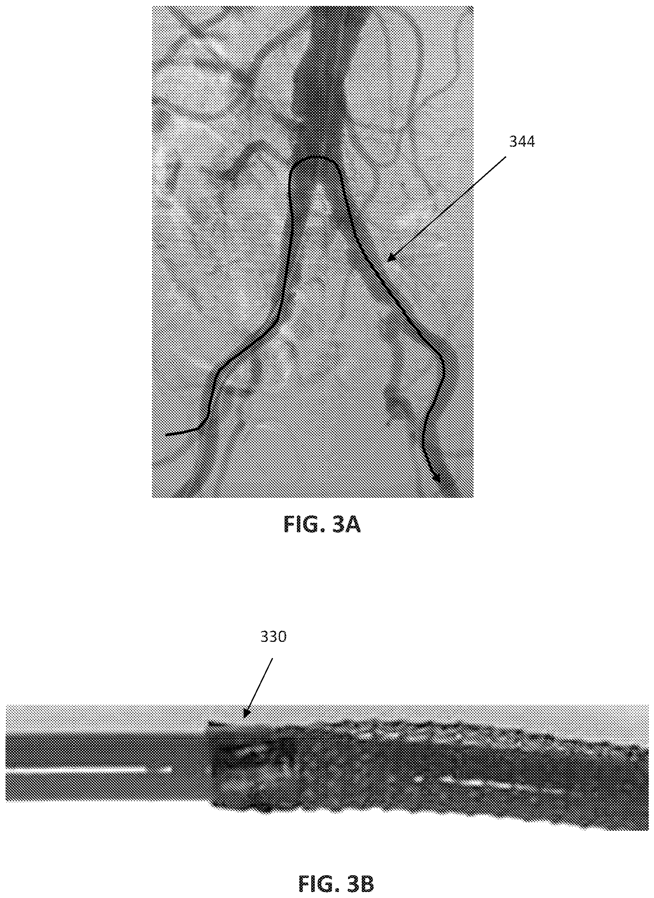

The tractor may lock up onto the outside of the inversion support catheter for a variety of reasons. For example, in some variations the tractor, particularly the knitted tractor, may experience increased friction on the outside of the catheter when navigating tortious regions of the vessel. For example, as shown in FIG. 3A, when navigating the Ilioa-Caval arch 344. This may cause the inversion support catheter diameter to ovalize, which may result in pinching of the tractor in this region. In some variations, the end of the tractor, which may include a cuff 330, as shown in FIG. 3A, may contribute to this issue, as it may stick to the catheter outer diameter, as shown in FIG. 3B. These issues may be mitigated in part by lubricating the cuff region (or using a lubricious material) and/or by enlarging the sheath or avoiding regions of high tortuosity. However, also described herein are more general solutions.

In particular, described herein is are tractors in which the knitted tube is configured to reduce the amount of stretch under tensions. For example, the tractors described herein have been shown to reduce or prevent locking down on the outside of the inversion support catheter when the knitted tractor is configured to stretch less than 3 percent when pulled in tension with a force of 2 Newtons (e.g., configured to stretch less than 2 percent when pulled in tension with a force of 2 Newtons). Although knitted tractors such as those shown in FIG. 2A-2B are generally highly stretchable, as the links are typically interlocking oval or circular links which may be formed of a filament of stainless steel, Nitinol, or a polymeric material, the percent of stretch may be limited by controlling the number of loops per turn (e.g., one circumference of the knitted tractor), and the diameter of the filament, relative to the circumference of the inversion support catheter. For example, a knitted tube formed of a filament that is knitted into a number of N loops per turn may be configured to stretch less than 3 percent when pulled in tension with a force of 2 Newtons based on the number of N loops per turn and a diameter of the filament. Other factors, such as the length of the loops and the filament material may contribute, but the number of loops and the cross-sectional diameter of the filaments may have the greatest effect on the stretch and therefore of the tractor under tension and therefore preventing locking down of the tractor over the outside of the inversion support catheter.

Note that there may be other ways to modify and/or reduce the amount of stretch of the knitted tractor, including stiffening the tractor (e.g., by adding or bonding a material, such as a sleeve, to the tractor). However, it may be undesirable to stiffen the tractor, and particularly the ability of the tractor to invert easily over the distal end opening of the inversion support catheter.

In general, any of the apparatuses described herein may also be configured so that they are biased (e.g., shape set, heat set, etc.) to prevent significantly reducing the inner diameter of the tractor in the un-inverted configuration when pulled in tension. Further, any of these apparatuses may include a lubricious material between the inversion support catheter outer surface and the tractor. Although such modifications may be beneficial, it has been found the limiting the stretch, by, e.g., by adjusting the number of loops of filament per turn based at least in part on the filament diameter and the outer circumference of the catheter may optimally allow both a highly flexible, low-friction movement of the tractor while optimally capturing material in a knitted apparatus.

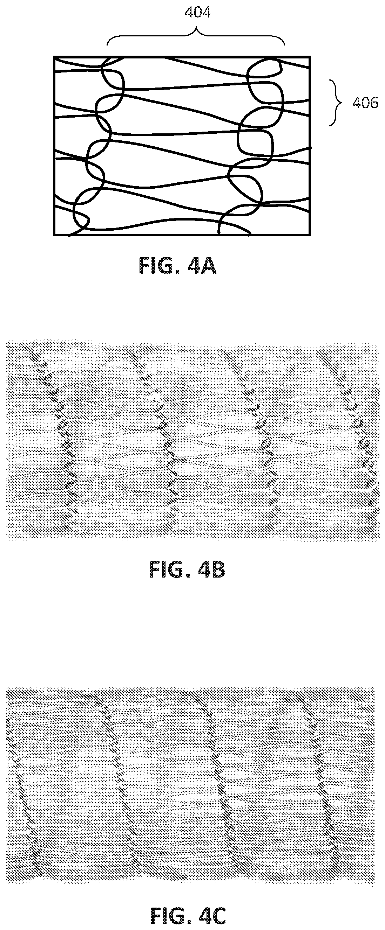

As shown in FIG. 4, a knitted tractor may include teardrop shaped loops each having a length per loop 400 and a width per loop 406. The ratio of the length per loop to width per loop may be greater than 6 (e.g., the length may be longer than six times the width), or in some variations greater than 7 times the length (e.g., greater than 8 times the length, 9 times the length, 10 times the length, etc.).

In general, the filament diameter and respective stiffness may be controlled to resist stretch. For example, the larger the wire diameter, generally the more resistant the knitted tractor may be to locking onto the outside of the catheter. However, larger diameter filaments may increase the stiffness of the tractor, which may risk damaging the vessel, particularly when inverting the tractor into the catheter. Larger stiffness may also increase the force required to invert the tractor, which may also be undesirable.

In some variations of the inverting tube apparatuses (e.g., thrombectomy apparatuses) the knitted tractor may be shape set to have an inner diameter that is much larger than the outer diameter of the inversion support catheter. See, e.g., U.S. Patent Application Publication No. 2019/0336148. However, in some variations it may be desirable for the knitted tractor to have an inner diameter that is closer to the outer diameter of the catheter, particularly where narrower-profile apparatuses are desired. These apparatuses may also have a lower pulling friction (e.g., require a lower pulling force) due to reduced friction within the catheter.

The number of loops per turn of the tractor may be controlled, e.g., by increasing or decreasing the number of needles used to form the knitted tractor tube. For example, FIG. 4B illustrates an example of a tractor tube formed with 22 needles per turn (e.g., having 22 loops per turn), while FIG. 4C shows an example of the same filament formed to the same inner diameter having 34 needles per turn (e.g., 34 loops per turn). In this example the filament is a 0.0012 inch (e.g., 0.03048 mm) (1 inch = 25.4 mm) diameter Nitinol wire configured to be used over a 5 French (1 French = 1/3 mm) inversion support catheter (e.g., for insertion into the body through a 6F tube), and the relaxed inner diameter of the tractor tube may be less than about 1 mm from the outer diameter of the inversion support catheter.

Different factors, including the filament material, filament diameter, number of loops per turn, length of the loops, filament material, and the circumference of the inversion support catheter were varied in a variety of longer (e.g., greater than 20 cm) tractors and examined to determine the locking force (e.g., the amount of force applied in tension) at which the tractor locked onto the outer diameter of an inversion support catheter of various dimension. The tractor may be assumed to be within 1.5 mm of the outer diameter of the inversion support catheter when at rest. Other criterion including the general force required to pull (and invert) the apparatus.

Table 1 shown in FIG. 5 shows some of these results, from which a multi-parametric analysis was performed. In FIG. 5, the number of loops per turn is shown for a variety of different wire thicknesses (shown in both inches and mm), and for a variety of different inversion support catheter outer diameters (shown in French and circumference of inversion support). The data shown in FIG. 5 is for Nitinol filaments. Similar results were found for other filament materials (e.g., stainless steel, polyester, nylon, expanded Polytetrafluoroethylene (ePTFE). Locking the locking forces for these various embodiments were either quantified (in g) or qualitatively determined (a for acceptable, u for unacceptable).

Surprisingly, the number of loops was identified as a critical variable, as was the diameter of the wire being knitted. Less significant were the loop dimensions (e.g., the ratio of length to diameter) and wire material. In addition to the data shown in FIG. 5, a percentage of stretch, when tensioned with 2 Newtons of force (as a standard) was applied, was determined for some of these examples, and the results were used to identify the minimum amount of stretch that could be made by the knitted tube in order to prevent or minimize locking down of the tractor onto the outside of the catheter (inversion support catheter) when the tractor tube is long (e.g., 20 cm or longer) where the inner diameter of the tractor rides close (e.g., within 2 mm, 1.5 mm, 1 mm, etc.) to the outer diameter of the inversion support catheter. Specifically, stretch less than 3 percent when pulled in tension with a force of 2 N avoids lock-down of tractors longer than 20 cm in the majority of cases, while still permitting the tractor to remain sufficiently flexible. This majority may be increase, e.g., when the percent is 2.9%, without unduly stiffening the tractor.

In some cases, the stretch profile for the knitted tractors may therefore depend on the factors discussed above (e.g., the number of loops per turn, the circumference of the inversion support catheter onto which the tractor is to be used, the diameter of the filament forming the loops, etc.). As shown in FIG. 5, a relationship between the number of loops per turn, the circumference of the support catheter (and therefore the circumference of the tractor tube configured to ride closely over the inversion support catheter) and thickness of the filament woven into the loops was identified and a threshold determined. Empirically, the square of the number of loops per turn times the diameter of the wire, divided by the circumference of the inversion support catheter may provide a unitless measure that may be used to determine if a particular tractor is appropriate for use in apparatuses in which the knitted tractor rides closely over the inversion support catheter in the relaxed state and is longer (e.g., 20 cm or longer).

As shown in FIG. 5, this cutoff is approximately 2.9 (e.g., greater than 2.9, greater than 3.0, etc.). This ratio of the length of the loops to width of the loops for these same apparatuses may be 6 or greater (e.g. 7 or greater, 8 or greater, 9 or greater, etc.). Below this cutoff and the locking force may be too low to permit use of the apparatus, which may otherwise jam onto the outer surface of the catheter and prevent it from sliding.

Thus, the relationship, which may be referred to as a choke ratio, is:

$$Choke\ ratio = \frac{N^2 \times D_{filament}}{C_{cath}} \qquad [1]$$

where N is the number of loops/turn of the tractor, $D_{filament}$ is the diameter of the filament (e.g., wire) forming the loops, and $C_{cath}$ is the circumference of the inversion support catheter on which the tractor is to be used.

The choke ratio is a unitless measure, and may be rearranged to provide a number of loops/turn for a particular filament and inversion support catheter. For example, the choke ratio may be used to solve for the minimum number of loops/turn in an apparatus in which the tractor rides closely over the inversion support catheter and is formed of a filament having a particular diameter. Surprisingly, this relationship shows that increasing the number of loops/turn results in increasing the resistant of the knitted tractor to collapsing and locking onto catheter OD when under tension. Increasing the number of loops also has been shown to have little effect on overall stiffness or pull friction, while increasing tensile strength.

For example, a knitted tractor for use with a five French (5 F) inversion support catheter, which may be introduced through a six French (6F) tube, may be formed of between 28-34 loops per turn of a 0.0012" (Nitinol) filament, to avoid locking up when pulled (e.g., with up to 200 g of force (1g of force = 0.00981 N)). The tractor tube is longer than 20 cm (e.g., may be 140 cm or longer) and may have a ratio of loop length/diameter of greater than 7, and may ride within 1 mm of the outer diameter of the inversion support catheter in a relaxed configuration.

In another example, a knitted tractor for use with a 9 French (9 F) inversion support catheter, which may be introduced through a 10 French (10F) tube, may be formed of between 24-32 loops per turn of a 0.0022" (Nitinol) filament, to avoid locking up when pulled (e.g., with up to 200 g of force). The tractor tube is longer than 20 cm (e.g., may be 140 cm or longer) and may have a ratio of loop length/diameter of greater than 7, and may ride within 1 mm of the outer diameter of the inversion support catheter in a relaxed configuration.

## Apparatuses Configured to Ingest Hard Materials

Also described herein are apparatuses that include an expandable funnel on the end of an inversion support catheter over which a tractor rolls to invert, and these funnels may be adapted or configured to assist in removing hard material (e.g., clots) that may otherwise prevent the tractor from withdrawing the hard material into the catheter.

Such apparatuses may be used with any tractor type (not limited to knitted tractors), including other woven tractors, laser-cut tractor tubes, etc. The tractor is typically arranged to invert over a distal end of the inversion support catheter at the expandable funnel so that an external portion of the tractor extends proximally over the inversion support catheter as the internal (inverted) portion of the tractor is drawn into the funnel, compressing and removing fluid from the material, e.g., clot, that is held by (e.g., grabbed by) the tractor, and pulling the inverted tractor into the inversion support catheter until the entire clot is captured. Although the use of a funnel on the end of an inversion support catheter may be particularly helpful in removing large (e.g., larger diameter) clots, in some cases, particularly for harder materials, the inner shape of the funnel has surprisingly been found to be important in the ability of the apparatus to compress and withdraw material into the inversion support catheter.

Described herein are apparatuses including inversion support catheters having funnels that are particularly well suited to grabbing and removing clot, and particularly large diameter clots, by using an inversion support catheter than includes an expandable funnel at the distal end. The collapsible/expandable funnel may be configured to operate with the compressive forces applied by pulling the flexible tube into the inversion support catheter so that it inverts into the inversion support catheter, capturing the clot. The collapsible/expandable funnel may be configured to assume a fully expanded, locked (e.g., "jammed") configuration when the flexible tube applies a laterally compressive force on the distal end face of the funnel. Further, the funnel may include openings (described herein as having a porous structure) through which fluid squeezed out of the clot may exit laterally out of the funnel as the clot is moved into the narrower-diameter lumen of the inversion support catheter and compresses the clot. For example, the openings through the collapsible/expandable funnel (which may be referred to herein as simply an expandable funnel) that permit fluid to laterally leave the funnel walls as the clot is compressed may also prevent clogging or jamming of the clot.

The interior of the funnels described herein may be shaped so that the material drawn into the funnel is held up within the funnel, allowing it to be broken down and compressed within the funnel without causing the tractor to jam, preventing pulling in the tractor and/or additional clot. Thus, described herein are methods and apparatuses in which the distal end region of the inversion support catheter is configured as a collapsible and/or expandable funnel. The expandable funnel apparatuses described herein may also or alternatively be adapted to prevent jamming of the tractor in the distal end opening of the funnel, including when the apparatus is used to remove large and/or hard clots. In some variations, these funnels may be adapted to include an inner wall within the funnel that divides the funnel into section, such as a small section (e.g., 30% or less, 25% or less, 20% or less, 15% or less, 10% or less, etc.) of the proximal (i.e., narrower) end of the funnel may have a steeper angle relative to the more distal end. In some variations the funnel inner lumen includes two or more chambers that are separated by a narrowing or constricted region. These adaptations to the funnel inner lumen may permit harder clots to be retained within the funnel as the tractor is pulled into and past the held-up clot, helping to break down the harder clots.

Any of the apparatuses described herein may be apparatus for removing material from a vessel that include an inversion support comprising a catheter having a catheter lumen and an elongate and flexible catheter body, and an expandable funnel disposed at a distal end of the catheter body. The funnel extends in a distal to proximal axis, wherein a distal end of the funnel defines a distal end opening in communication with an interior of the funnel and the catheter lumen, respectively.

Funnels that may help break apart and compress harder materials may include two or more regions within the interior of the funnel lumen. For example, in some variations the open configuration the interior of the funnel may have a wall angle of less than 14 degrees relative to the proximal to distal axis for a majority (e.g., > 50%, or in some variations >55%, greater than 60%, greater than 65%, greater than 70%, greater than 75%, greater than 80%, etc.) of a length of the funnel and may include a first region in which the wall angle is between 14 and 50 degrees with respect to the distal to proximal axis. The apparatus also typically includes a tractor comprising a flexible tube that extends distally in an un-inverted configuration along an outer surface of the catheter, inverts over the distal end opening and extends proximally within the catheter lumen in an inverted configuration. The flexible tube may be configured to invert by rolling over the distal end opening when a first end of the tractor is pulled proximally within the catheter lumen.

FIG. 6 illustrate an example of an inverting tube apparatus 601 similar the one shown in in FIG. 1A. In this example, the inverting tube apparatus is in a large vessel that has a clot with a diameter that is greater than 2x the inner diameter of the inversion support catheter. Although the clot may be ingested and removed by the inverting tube apparatus 601 having a narrower-diameter inversion support catheter, the efficiency of the inversion may be low, e.g., requiring a flexible tube having approximately many times longer than the length of the clot tube to capture the entire clot. Capture may be even further complicated if the clot is or includes hard material (including calcified material, etc.). FIG. 6 illustrates one problem addressed by the apparatuses described herein; specifically, how to efficiently large diameter and/or hard clots into relatively smaller diameter inverting tube apparatuses. In this example, while a 10 mm or larger diameter clot may be pulled into a 3 mm diameter (e.g., 8 French) inversion support catheter of the inverting tube apparatus, the efficiency may be low, because the length of the flexible tube that must be used to capture the entire length of the clot may be extremely high. In some cases, it may not be possible to engulf the clot where the clot comprises a hard material that may be difficult to compress. The inverting tube apparatuses described herein may address and improve this efficiency of operation in a number of ways.

In particular, the methods and inverting tube apparatuses described herein may dehydrate the clot as it is drawn into the inverting tube apparatus. Clots, including even hard or partially calcified clots, may include a large amount of fluid that may be compressed and removed by the inverting tube apparatuses described herein. For example the flexible tube is typically porous, and may be, for example, a woven and/or knitted material. In addition, in some variations the distal end region of the inversion support tube may be configured, particularly at the distal end region (e.g., the distal 5 mm, distal 4 mm, distal 3 mm, distal 2 mm, distal 1 mm, distal 0.9 mm, distal 0.8 mm, distal 0.75 mm, distal 0.7 mm, distal 0.6 mm, distal 0.5 mm, distal 0.4 mm, etc.) may be porous to allow fluid to escape laterally out of the inversion support catheter from the clot as the clot is drawn into the elongate inversion support catheter, so that the cot may compress more efficiently, rather than elongate or stretching. In particular, described herein are apparatuses and methods that include a funnel-shaped distal end on the inversion support catheter that may be porous (particularly at the region near the base of the funnel) to allow compression of the clot material and ejection/removal of fluid from the clot laterally out of the sides of the inversion support catheter as clot is drawn proximally into the inversion support catheter by the rolling of the flexible tube (e.g., tractor). The funnel may be expandable (also referred to herein as collapsible) and may be integral with or attached to the distal end of the inversion support catheter. The funnel may be collapsed and introduced through a sheath/guide catheter (e.g., an intermediate catheter), so that it may fit, in a collapsed state, into a 6 French, 8 French, 10 French, 12 French, 14 French, 16 French, 28 French, 20 French, and/or 24 French sheath. The expandable funnel may be self-expanding. Alternatively or additionally, the expandable funnel at the distal end of the inversion support catheter may be expanded by actuation of the flexile tube; e.g., pulling the flexible tube into the inversion support catheter proximally to roll the flexible tube over the distal end of the inversion support catheter may apply a proximally-directed compressive force that pulls and expands the expandable funnel. The funnel may have a maximum outer diameter that is greater than 2x (e.g., greater than 2.5x, greater than 3x, greater than 3.5x greater than 4x, greater than 4.5x, greater than 5x, etc.) the maximum outer dimeter of the collapsed configuration; the maximum outer diameter of the funnel in the collapsed configuration may be approximately the same as, or slightly larger than, the maximum outer diameter of the body region of the inversion support catheter (e.g., 1x, 1.01x, 1.1x, 1.2x, etc. the outer diameter of the proximal portion of the inversion support catheter). In some variations the funnel has an outer diameter of between 2-26 mm.

In any of these variations, the flexible tube may also be adapted to better engulf and compress large-diameter clots. For example, the flexile tube in the un-inverted configuration when outside of the inversion support catheter (e.g., in the vessel) may have an outer diameter that is selected to be approximately the same as or larger than the maximum outer dimeter of the open configuration of the funnel. An expandable funnel may allow the flexible tube (e.g., woven tractor) to grab clot at edges of cross section rather than the center of clot, which may enable a more efficient clot ingestion. However, in some variations the funnel, and particularly those having a low wall angle (e.g., of 14 degrees of less) may have difficultly handling hard material (e.g., calcified material).

In flexible tube variations (e.g., tractor variations) described herein, it may be beneficial to have the expanded non-inverted outer diameter of the flexible tube (e.g., the portion of the flexible tube on the outside of the inversion support catheter prior to being pulled into the catheter and inverted) be heat-set to a larger diameter (OD) than the maximum outer diameter of the expanded funnel, and preferably as large as possible with respect to the clot OD. Larger OD flexible tube may have higher efficiency for grabbing and compressing clot. This may be independent of whether there is a funnel on the distal end of the inversion support catheter. For example, for a flexible tube (e.g., tractor) formed of a woven material, the OD of the un-inverted flexible tube may be selected to be at least 1/3rd of the clot OD (or vessel ID), for example, the expanded, un-inverted flexible tube may have an OD that is greater than or equal to about 50%, 60%, 70%, 80%, 90%, 100% or 110% of the clot OD (or vessel ID).

The distal end of the inversion support catheter, and particular an expandable funnel on the distal end, may be porous. The ability to allow fluid from compressed clot to exit from out of the sidewalls of the funnel inner diameter (e.g., lateral to the walls of the inversion support catheter, rather than just from the distal and proximal ends) may provide a place for the fluid removed from the clot to go and may improve the efficiency of the apparatus, allowing for much shorter flexible tubes to remove a comparable length of clot. If the clot is not allowed to escape laterally (e.g., when using a non-porous funnel), the fluid removed may build up at the base of the funnel and may reduce the clot efficiency. Thus, in some variations, the funnel is porous or at least partially porous, e.g., near the base of the funnel, where the compressive ratios of the clot are the highest.

In any of the variations described herein, the inversion support catheter may be relatively large, so that the clot does not have to be compressed as much. In the peripheral vasculature, for example, the inversion support catheter may have an outer diameter that is greater than 1 mm, e.g., greater than 1 mm, 1.2 mm, 1.4 mm, 1.5 mm, 2 mm, 2.5 mm, 3 mm, 3.5 mm, 4 mm, 4.5 mm, 5 mm, etc.

In general, any of the apparatuses described herein may also increase the efficiency of the apparatus for removing clot by reducing the force need to remove the clot. For example, the methods and apparatuses described herein may include a lubricious material on the distal end (e.g., the funnel) of the inversion support catheter. For example, in any of these apparatuses the funnel may be lined with a slippery material (e.g., a PTFE liner) that may produce a lower ingestion pull force and/or may reduce the ingesting efficiency. Slippery funnels may allow a clot mass to be drawn into the mouth of funnel rather than pulling it into the catheter.

In some variations the funnel may be configured to have a specific shape (e.g., taper) that may also assist in increasing the efficiency for compressing and/or dehydrating the clot and may help reduce the amount of force required. For example, in some variations, longer funnels may have a lower ingesting forces and better clot ingesting efficiency compared to shorter funnels of the same maximum OD/minimum ID. Examples of funnels may have a maximum OD of, e.g., 3 mm, 5 mm, 6 mm, 8 mm, 10 mm, 12 mm, 15 mm, 20 mm, 25 mm, etc. Exemplary funnel lengths may be, for example, 5 cm, 10 cm, 15 cm, 20 cm, 25 cm, 30 cm, 40 cm, 50 cm, 50 cm, 100 cm, etc. The body portion of the elongate flexible inversion catheter may be, e.g., a 3 French (F), 4 F, 5 F, 6 F, 7 F, 8 F, 9 F, 10 F, 11 F, 12 F, 14 F, 16 F, 18 F, 20 F, 25 F, etc., catheter.

For variations in which the flexible tube comprises a woven material, coarser waves may have increased efficiency. For example, a larger number of weave "fingers" (e.g., loops) in the transverse direction of the tube may (per weave circumference) may have a greater clot ingesting efficiency. For example, the number of grabbing fingers may be at least 10, 20, 20, 40, 50, 60, 100, etc., per tubular weave circumference.

In general, the inverting tube apparatuses described herein may be highly flexible, both before actuating and during operation. For example, the flexible tube (e.g., tractor) may not significantly increase the stiffness/flexibility of the catheter of the elongate inversion support, and particularly the distal end region of the catheter, to avoid impacting maneuverability. Described herein are flexible tractor tube portions that increase the stiffness of the last y cm (e.g., distal most 20 cm, 18 cm, 15 cm, 12 cm, 10 cm, 9 cm, 8 cm, 7 cm, 6 cm, 5 cm, 4 cm, 3 cm, 2 cm, 1 cm, etc.) of the catheter less than a predetermined percentage (e.g., less than 10%, 12%, 15%, 18%, 20%, 25%, 30%, etc.). For example, described herein are flexible tractor tube portions that pass through the catheter and double back over the distal end of the catheter but increase the stiffness of a distal 5 cm of the catheter by less than 15% of the stiffness of the distal 5 cm of the catheter without the flexible tube extending therethrough and doubling back over the distal end of the catheter.

As mentioned, the flexible tube (e.g., tractors) may be woven, braided and/or knitted materials. For woven and braided materials, which may include a plurality of fibers that are woven or braided to form the inverting tube, these structures may be tuned to prevent jamming and/or to reduce the force necessary to pull the tractor and invert over the catheter tip. For example, the mechanical atherectomy apparatus may include a knitted or braided flexible tubes that can roll freely around the tip of catheter even in a tortuous anatomy and when grabbing clot by tuning one or more of the braid structure; minimizing the braid angle; including a hydrophilic coating on the distal aspect of the catheter outer diameter (OD) or the inner diameter (ID) of the braid (e.g., tractor); including a radiused wall on the catheter; and/or increasing the stiffness of the distal tip region relative to adjacent proximal regions. Alternatively, it may be advantages to have a hydrophilic coating on 1, 3, 5, 10, or 15 cm of the distal ID, or even on the entire catheter ID.

As mentioned, the flexible tube (e.g., tractor) may be braided, woven, knitted, etc., and may be configured to collapse down into the inner diameter (ID) of the catheter as little as possible. For example the tractor may collapse to an ID that is greater than, equal to, or within 90%, 85%, 75%, 70%, 65%, 60%, or 50% of the catheter inner diameter (ID)/Catheter Tip OD, since, where this ID is based on the elongate body region of the inversion support catheter, when the tractor is being pulled around catheter tip it may create axial tension on the tractor (e.g., braid, knit, etc.) that may otherwise inadvertently and undesirably cause the tractor to jam on the catheter tip. When tractor is pulled around catheter tip, the tractor may be pulled in the axial orientation creating axial tension on tractor structure as the tractor is being pulled through the catheter ID. By having the tractor elements jam at an ID greater than or equal to 90%, 85%, 75%, 70%, 65%, 60%, or 50% of the catheter ID (or in some variations, OD), when being axially tensioned, the tractor is less likely to grab/synch down onto the catheter tip, helping the braid roll around the catheter tip with less axial force applied by the user. If less axial force is required by the user to pull the tractor structure around the tip then the catheter tip is less likely to buckle or deflect when retracting the tractor. It may be advantageous to minimize the chance the catheter tip will buckle. The tractor can be tuned to "jam" at a specific ID by controlling any of the following variables and in any combination: selecting a specific number of braid ends, selecting the size/diameter of the braid ends; selecting the braid material (e.g., multifilament or monofilament); heat setting the bias on the braid (e.g., braid diameter); and selecting a braid pattern, e.g., 1x2, 1x1 or any other pattern.

The braid angle may be minimized to prevent locking up of the rolling of the tractor over the catheter end opening. Typically, the lower the braid angle (e.g., 45 degrees or less, 40 degrees or less, 35 degrees or less, 30 degrees or less, 25 degrees or less, 20 degrees or less, etc.) the less likely it is to have the braid cross over points catch on the catheter tip.

In any of the variations described herein, the catheter and/or a surface of the tractor may be coated to enhance rolling over the distal end region of the catheter. It may be helpful to have a hydrophilic coating on the distal aspect of the catheter OD or the ID of the tractor so the tractor can more easily side over the catheters distal end and around the tip of the catheter when pulled through the inside of the catheter.

The stiffness of the distal of the elongate inversion support catheter may be sufficiently stiff to prevent collapse as the tractor is pulled; it may also be lubricious (e.g., by a coating or material property). The distal most section of the elongate inversion support catheter tip (e.g., the last 5 mm) may be fabricated of a material which is stiff enough and lubricious enough so the distal tip of the catheter does not collapse or buckle inward ward when the braid structure is rolling around the catheter tip. Thus, the distal tip may have a stiffness that is greater than the more proximal region at the distal end of the catheter.

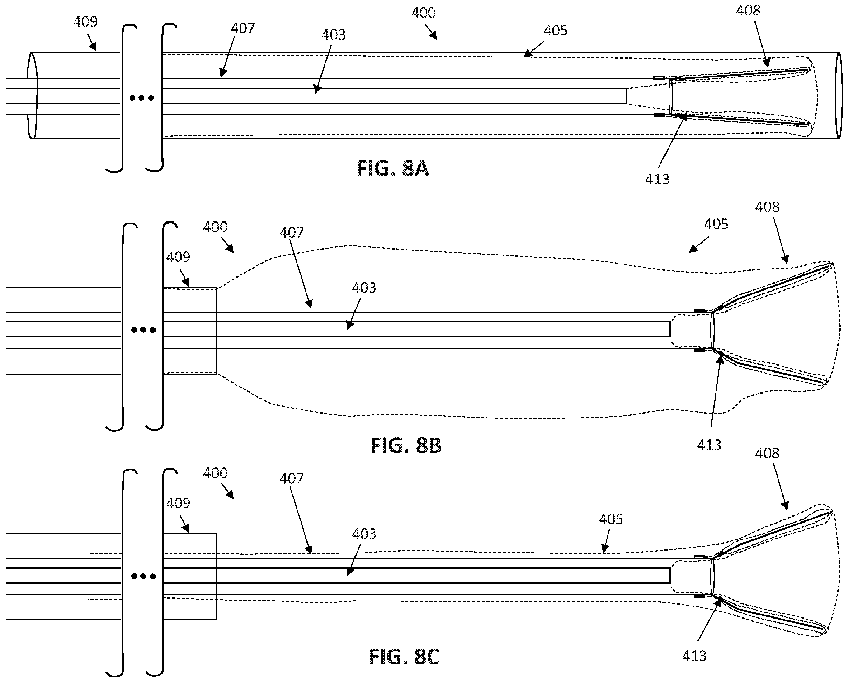

FIGS. 7A-7B and 8A-8B illustrate examples of inverting tube apparatuses that each include a funnel region at the distal end of an inversion support catheter. In this example the funnel includes two regions; a first region 350 having a wall angle of less than 14 degrees that includes >60% of the inner wall of the funnel, and second more proximal region 351 having a wall angle of >15 degrees that extends for about 30% of the inner wall of the funnel. Thus, FIG. 7A shows a first variations of an inverting tube apparatus 300 that includes an elongate, flexible inversion support catheter 307 that has an expandable funnel 308 at the distal end, shown in a collapsed configuration in FIG. 7A within an intermediate (e.g., delivery) catheter 309, and in an open configuration in FIG. 7B after being released from the intermediate catheter. The funnel may be formed of a woven material and may be porous, particularly at the base region 313, where the funnel extend from the body of the elongate body of the inversion support catheter. A flexible tube 305 extends over the distal end (including the funnel) of the inversion support catheter and inverts over the distal opening of the funnel. The flexible tube may be, e.g., a knitted material, and may be biased to expand to an outer diameter (OD) that is larger than the OD of the funnel 308 in the open configuration. The flexible tube is attached to a distal end region of a puller 303. In the example shown in FIGS. 7A-7B the puller extends distally 315 further than the distal end of the funnel, as shown. Although the flexible tube (e.g., tractor) is attached to the distal end region of the puller, the end of the flexible tube in this example is attached proximally of the distal end of the device.

In the example of an inverting tube apparatus 400 shown in FIGS. 8A-8B, the flexible tube 405 is attached at the distal end region of the puller 403 closer to or at the distal end of the puller. FIG. 8A shows the inverting tube apparatus 400 within an intermediate catheter (e.g., deliver catheter) 409 with a funnel 408 at the distal end of the inversion support catheter 407 within the intermediate catheter in a collapsed configuration. The funnel may include one or more (e.g., a plurality of circumferentially-arranged) openings or pores at the base 413 region to permit fluid from the clot to exit the inversion support catheter as the clot is pulled into the inversion support catheter by the rolling flexible tube 405 (e.g., tractor region). FIG. 8B shows the apparatus at least partially deployed from out of the intermediate catheter 409, with the expandable funnel 408 expanded. As in FIGS. 7A and 7B the funnel in FIGS. 8A-8B includes two regions having different wall angles.

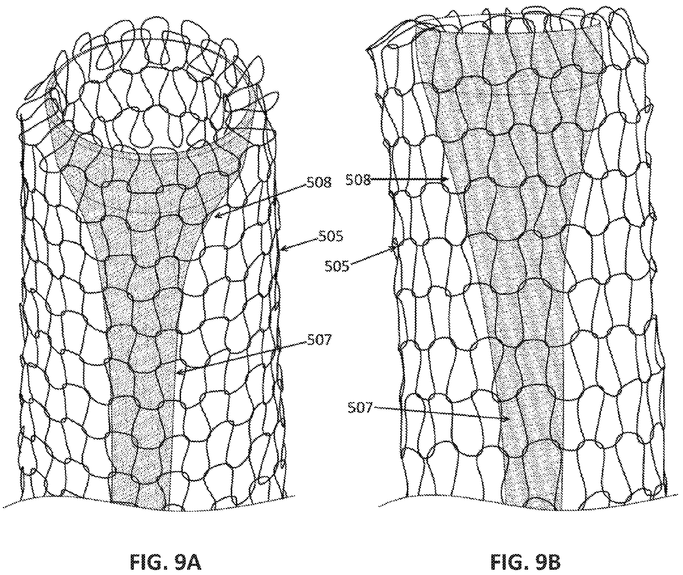

FIG. 8C is an example of another apparatus 400' in which the inversion support catheter 407' includes an expandable funnel 408' (shown expanded) having multiple regions (with different wall angles) within the funnel lumen. As in FIGS. 8A-8B, the flexible tractor 405' may roll and invert to capture clot material when used as a thrombectomy device. In FIG. 8C the flexible tube may be a knitted tube, forming a plurality of loops or fingers at the distal-facing end of the apparatus, which may help capture clot material. This is shown in more detail in FIGS. 9A and 9B. In this example, the tractor is configured (as described above) to more closely match the outside of the inversion support catheter, as shown.

In FIG. 9A and 9B, an example of a porous funnel 508 forming the distal end of an inversion support catheter 507 is shown with a knitted flexible tube 505 shown inverting over and into the funnel when the flexible tube is pulled proximally into the funnel. As described herein, the inside of the funnel (the funnel lumen) may be configured to have different wall angles, and/or may include one or more constricted regions. The inner wall angles of the funnel may be different from the outer wall angle of the funnel.

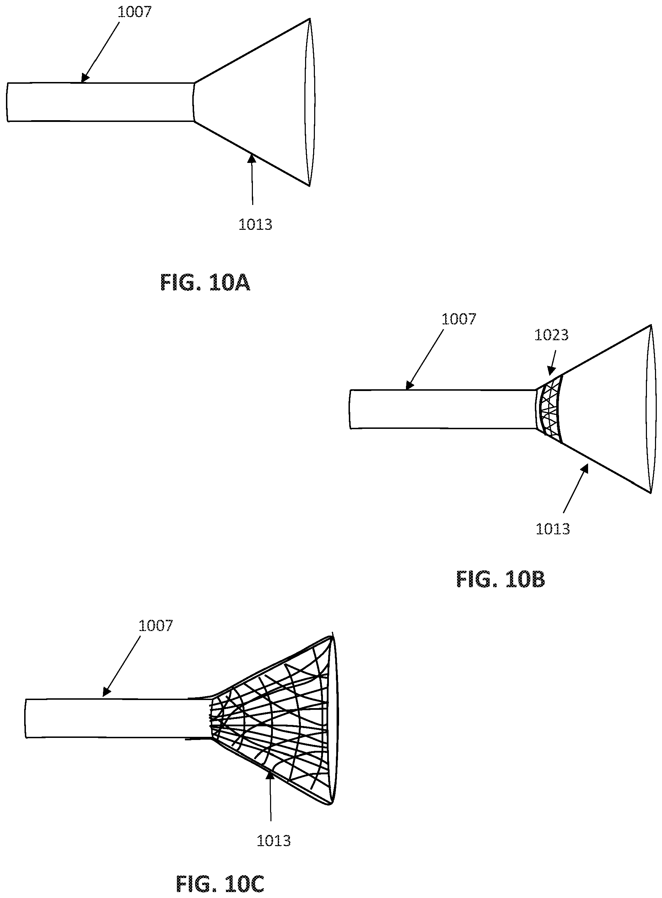

Any appropriate expandable funnel-shaped distal ends may be used. For example, FIGS. 10A-10C illustrate different variations of funnel-shaped distal ends having differently shaped outer surfaces. In FIG. 10A the funnel 1013 may be solid or may include openings for allowing the passage of fluid (e.g., may be porous), including porous over its entire surface or a portion of the surface. FIG. 10B shows an example in which only a portion of the expandable funnel is porous. In FIG. 10B, the base region 1023 is porous, and includes a plurality of openings arranged circumferentially around the perimeter of the base region of the funnel 1013.

The expandable funnel 1013 variation shown in FIG. 10C is porous over its entire length, and is shown formed of a woven material (e.g., a metal or polymeric fiber) that may be doubled back over itself to form the funnel-shape, and include the flexible inversion support catheter 1007.

FIGS. 11A-11D illustrate one example of a distal end of an inversion support catheter including a funnel. In this example, the funnel is formed integrally with the body of the inversion support catheter. As shown by the schematic in FIG. 11A, the funnel shape includes a framework formed by a plurality of fingers or struts 1111 formed, e.g., by cutting (e.g. laser cutting) the distal end of the body of the inversion support catheter 1107. In FIG. 11A a braided or woven funnel body 1118 is attached to the struts; the woven body is attached 1105 to the body of the inversion support catheter on one end, and on the other end 1109 to lock it in place over the struts. FIG. 11B shows an example of a body of an elongate support catheter that has been cut into a plurality of struts or fingers over which the funnel body may be supported, as shown in FIG. 11C. The distal end of the funnel 1131 is opened and may extend beyond the struts and may jam to form a locked open configuration having a high compression strength, even in the absence of the underlying struts or fingers. FIG. 11D shows an example of an open distal end of the funnel of the apparatus.

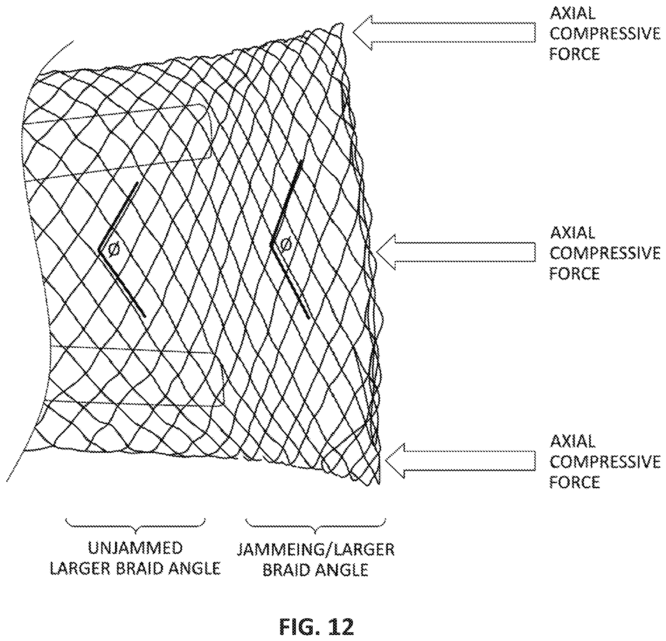

FIG. 12 is an enlarged version of the distal end of the funnel shown in FIG. 11C, illustrating the application of axial compression that may cause the distal end of the apparatus to 'jam' and from a larger braid angle (Ø) at the distal end, compared to the unjammed more proximal end. This compressive force may help open (and hold open) the expandable funnel.

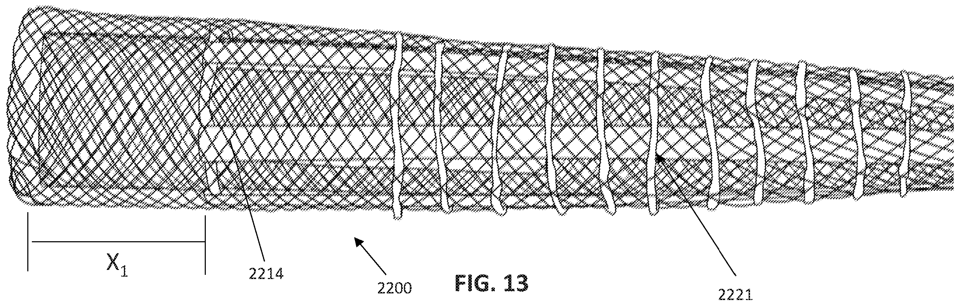

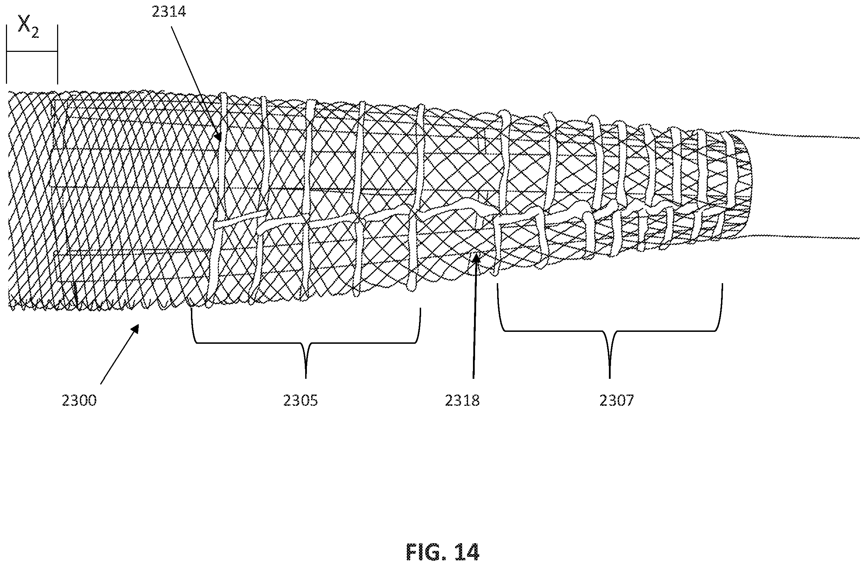

FIGS. 13-14 illustrate a funnel 2200 formed by applying the braded wall material onto the tines at the distal end of the inversion support catheter. The inner and outer walls of the woven material forming the funnel may be stitched together by a suture 2221 that is arranged radially around the funnel and may constrain it from expanding further, as described above. The tines may slide axially relative to the inner and outer walls. In any of the funnels described herein the mesh material forming the inner and outer walls may extend distally further than the distal end of the tines. In the relaxed configuration the braid length distal to the distal ends of the tines 2214 is shown as a distance $x_1$ mm. FIG. 14 shows the funnel with the braid jammed 2213, as it would be if the tractor (e.g., the flexible tube) were loaded over the funnel and pulled proximally into the inversion support catheter. In this configuration the axial length of the braided wall extending beyond the tip of the tines as $x_2$ mm.

In some variations it may be beneficial to limit the axial length of the braided wall extending beyond the tip of the tines in the fully expanded (e.g., jammed) configuration. This may prevent instability, and particularly lateral instability. For example, it may be beneficial to limit the axial length of the braided wall extending beyond the tip of the tines in the fully expanded (e.g., jammed) configuration to 10 mm or less (e.g., 8 mm or less, 7 mm or less, 6 mm or less, 5 mm or less, 4 mm or less, 3 mm or less, 2 mm or less, such as between 1 mm and 10 mm, between 1 mm and 8 mm, between 1 mm and 7 mm, between 1 mm and 6 mm, between 1 mm and 5 mm, between 1 mm and 4 mm, etc.). In particular, it may be beneficial to limit it to 5 mm or less.

Any of the apparatuses described herein may be adapted so that the funnel may more easily compress hard materials. For example, in some variations the apparatus may be configured so that the funnel includes a bulge or larger-diameter region (e.g., a region having a higher wall angle, such as between 15-50 degrees) near the proximal end of the funnel. This may be formed by, e.g., modifying the suturing of the mesh forming the funnel inner wall. In some variations the funnel may have a length of between 15-40 mm (e.g., between 15-35, between 15-30, between 15-25, between 15-20, etc.). In some variations, the funnel may be configured to reduce the cross-sectional area within the funnel in all or a region of the inner lumen of the funnel. In some variations one or more projections that may help break up the harder materials (e.g., clots) may be present in the funnel. These projections may be configured to project into the lumen of the funnel, but may allow the tractor to move over and/or around the projections.

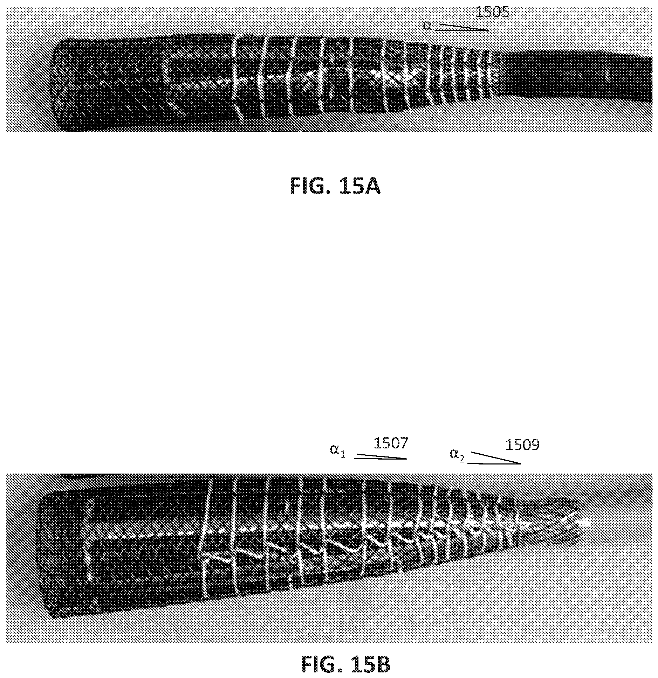

FIGS. 15A-15B illustrate example of funnels, showing the external view. In FIG. 15A a funnel having a very narrow outer profile (e.g., a maximum external wall angle, $\alpha$ 1505, of 7 degrees or less) is shown. FIG. 15B shows an example having an initial (proximal) external wall angle, $\alpha_2$ 1509, that is approximately 15 degrees for the first 25% of the length of the expanded funnel, then having an external wall angle, $\alpha_1$ 1507, of less than 10 degrees for the rest of the length of the expanded funnel.

FIGS. 16A-16D show examples of profiles of funnels having two or more regions adapted to have different internal wall angles. For example, in FIG. 16A the apparatus includes an initial, proximal region of the inner lumen of the funnel having a wall angle $\beta$ 1613 that is approximately 14 degrees (or more) over this initial region 1605, and a more distally located region 1607 having a wall angle 1611 that is 10 degrees or less. For example, in FIG. 16A, the region of the funnel lumen 1604 having a wall angle that is less than 14 degrees ($\alpha$ 1611) is approximately 65% of the length of the funnel, while the region having a wall angle ($\beta$ 1613) that is 14 degrees or larger is approximately 35% of the length of the funnel, in the distal to proximal axis. The outside of the funnel is continuous and may have a contour that is different from the inner lumen of the funnel; for example, the outer wall angle may be less than 14 degrees over the entire length, as shown.

Similarly, in FIG. 16B, the apparatus includes an initial, proximal region of the inner lumen of the funnel having a wall angle $\beta$ 1613' that is approximately 30 degrees over this initial region 1615, and a more distally located region 1617 having a wall angle 1611' that is 10 degrees or less. For example, in FIG. 16B, the region of the funnel lumen 1604' having a wall angle that is less than 14 degrees ($\alpha$ 1611') is approximately 75% of the length of the funnel, while the region having a wall angle ($\beta$ 1613') that is 30 degrees is approximately 25% of the length of the funnel, in the distal to proximal axis. The outside of the funnel is continuous and may have a contour that is different from the inner lumen of the funnel; for example, the outer wall angle may be less than 20 degrees over the entire length, as shown.

In FIG. 16C, the apparatus includes an initial, proximal region of the inner lumen of the funnel having a wall angle $\beta$ 1613' that is approximately 50 degrees over this initial region 1625, and a more distally located region 1627 having a wall angle 1611" that is 7 degrees or less. For example, in FIG. 16C, the region of the funnel lumen 1604" having a wall angle that is less than 7 degrees ($\alpha$ 1611") is approximately 75% of the length of the funnel, while the region having a wall angle ($\beta$ 1613'') that is 50 degrees is approximately 25% of the length of the funnel, in the distal to proximal axis. The outside of the funnel is continuous and may have a contour that is different from the inner lumen of the funnel; for example, the outer wall angle may be less than 30 degrees over the entire length, as shown.

FIG. 16D illustrates another example in which the inner lumen of the funnel has three regions with different wall angles, as shown. In FIG. 16D, the apparatus includes an initial, proximal region 1639 of the inner lumen of the funnel having a wall angle $\alpha_2$ 1612 that is approximately 10 degrees over this initial region 1639, a central region 1635 having a funnel wall angle β1 1613‴ that is approximately 14 degrees (or more), and a more distally located region 1637 having a wall angle 1611‴ that is 10 degrees or less. The combined length of the regions of the funnel lumen 1604‴ having a wall angle that is less than 14 degrees (1637 and 1639) is approximately 75% of the length of the funnel, while the region 1639 having a wall angle that is 30 degrees is approximately 25% of the length of the funnel, in the distal to proximal axis. The outside of the funnel is continuous and may have a contour that is different from the inner lumen of the funnel; for example, the outer wall angle may be less than 20 degrees over the entire length, as shown.

In FIGS. 16A-16D the wall angle of the inner lumen of the funnel is measure to determine the acute, distally-facing angle, between the distal-to-proximal axis 1608. Any of these variations may be configured as described above, forming of internal arms or tines that are bent or otherwise configured to form the inner wall angles described and covered in a mesh (e.g., woven or knitted mesh).

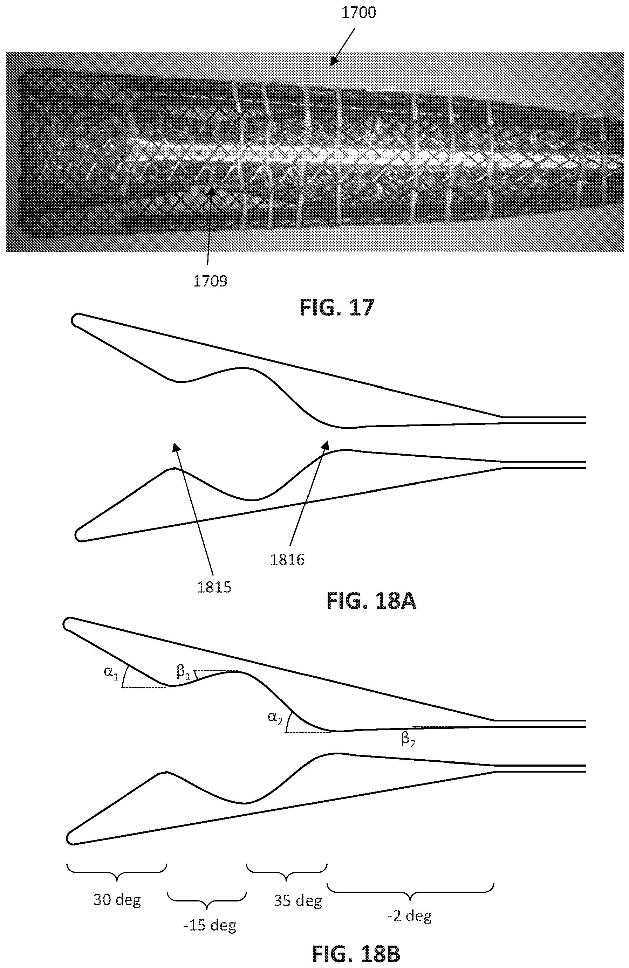

In some variations, the funnel may be configured so that the inner lumen include one or more constricted regions within the funnel. This is illustrated in FIGS. 17-18B, for example. This configuration may also allow the apparatus to capture and remove various material having various degrees of firmness (e.g., soft to hard). For example, in FIG. 17 the prototype funnel is formed at the end of an inversion support catheter by forming tines or arms over which a mesh is attached from a woven material. In this example the inner lumen of the funnel includes two constricted regions within the funnel lumen inner diameter (ID). By controlling the shape of the funnel inner diameter, the path the tractor follows when inverting into the funnel may also change, which may improve the ingestion characteristics of the apparatus.

In FIG. 17, the mesh forming the inner lumen of the funnel 1700 has been constricted in a medial region 1709 to form a single reduction in the funnel inner diameter. This may be accomplished, for example, by a ring, suture, or other element limiting the expansion of the wall in this region. FIGS. 18A-18B illustrate another example of a funnel (shown in section) showing two constricted regions. In this example, the first constricted region 1815 may be formed as described above, and includes a first region having a wall angle ($\alpha_1$, shown in FIG. 18B) that is approximately 30 degrees, adjacent to a second region having a negative wall angle (e.g., $\beta_1$) that is approximately -15 degrees and a third region having a wall angle that is approximately 35 degrees ($\alpha_2$) and a most proximal region having a wall angle of approximately -2 degrees ($\beta_2$). Thus, the interior of the funnel includes two constrictions forming three continuous regions into which material may be drawn. In some variations, the reduction in the funnel ID may measure between 10 to 90% of the largest funnel ID. The inner diameter reduction may be positioned at a discrete location between the base and the top of the funnel. Similarly, multiple inner diameter constructions or reductions within the lumen of the funnel (e.g., two or more) may each measure between 10 to 90% of the largest funnel inner diameter, and may be positioned at a discrete locations between the base of the funnel (at the connection to the catheter lumen) and the distal open end of the funnel.

As discussed above, any of these funnels may include a porous structure to enable clot or tissue to be partially desiccated when pulled into through base of funnel, by allowing fluids to ooze out through the side of the funnel. The funnels described herein may have a smooth transition from the funnel ID to the catheter ID. This may be achieved by laser cutting the tines at the distal of the catheter, as shown. The porous structure and/or the smooth transition may also be provided in these examples by the porous metallic mesh (e.g., braid) structure forming the walls.

The inversion support catheters described and illustrated herein may be adapted to prevent collapse, even when force is applied by the flexible tube either without or with a clot material. In any of these variations, the funnel needs to be able to handle axial loads (e.g., loads applied along axis of catheter shaft length) that may be in excess of 1, 2, 3, 4, 5, 10, 15 and/or 20 ⁻kg (1 kg = 9.81 N), without collapsing, e.g., when there is resistance ingesting the clot, while still allowing the flexible tube (e.g., tractor) to roll around the top of the funnel and into the inversion support catheter. Axial stiffness may be achieved at least in part by configuring the braided wall of the funnel have a jammed configuration at tip as described above. Axial stiffness may also be improved by limiting the length of the braided wall extending beyond the distal tips of the tines in the jammed configuration (e.g., to 5 mm or less). In some configuration, axial stiffness may also be improved by including the circumferential support (e.g., filament) between the tines, as described above, which may distribute the load exerted from the tractor on the funnel tip, so that the funnel tip remains round and no one finger gets isolated and collapses.

In general, these same factors may improve the radial stiffness as well. The end of the funnel may also preferably be sufficiently stiff to prevent the funnel from collapsing radially when the tractor rolls around the tip. Radial stiffness of funnel may be achieved at least in part by configuring the braided wall of the funnel have a jammed configuration at tip as described above. Radial stiffness may also be improved by limiting the length of the braided wall extending beyond the distal tips of the tines in the jammed configuration (e.g., to 5 mm or less). In some configuration, radial stiffness may also be improved by including the circumferential support (e.g., filament) between the tines, as described above, which may distribute the load exerted from the tractor on the funnel tip, so that the funnel tip remains round and no one finger gets isolated and collapses.

Thus, in any of the funnels described herein, the funnel may be formed of a plurality of tines or arms over which a mesh forming the inner and/or outer walls is attached. As mentioned briefly above, it may be beneficial to allow the mesh to extend and collapse relative to the tines, so that they may assume a jammed angle, holding the funnel open when force is applied by pulling the tractor into the funnel distal end opening.

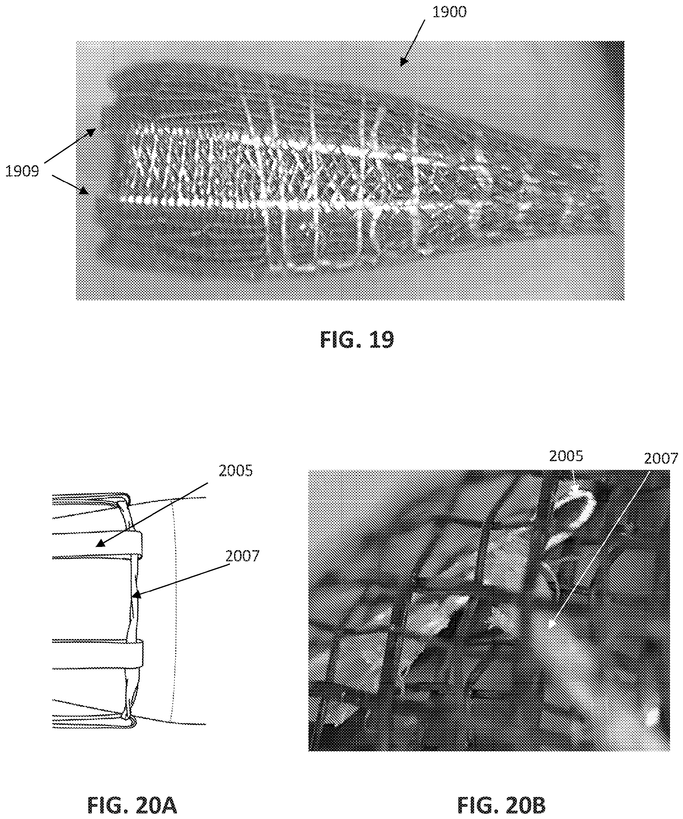

In such variations, however, it may be important to keep the tines from extending distally out of the mesh, as shown in FIG. 19. In the example funnel 1900 shown in FIG. 19, the tines have projected out of the distal end of the funnel 1909. This may result in one or more regions in which the tractor (which may be, e.g., a knitted material) may catch or snag. Thus, it would be desirable to prevent the tines from projecting out of the distal end of the funnel. As shown in FIG. 20A, this may be achieved by the use of one or more bridging elements 2007, such as a suture (e.g., a restraining filament) integrated between the tines of the funnel to prevent the tines 2005 from extending distally out of the braided material forming the walls of the funnel. In this example, the suture may limit the tines of the funnel from extending out of the woven material forming the outer walls of the funnel.

The restraining filament(s) may be referred to herein as circumferential supports extending radially around the funnel surface and constraining the maximum outer diameter of the expandable funnel. In variations in which the restraining filament is held in place between the bent-over tines (as shown in FIGS. 20A-20B), the restraining filament must be proximally constrained to prevent it from sliding back (proximally) and allowing the tines to project into the mesh, as shown in FIG. 20B. Because the tractor may compress (and open) the funnel distally with a significant amount of force, in the absence of a proximal constraint, the restraining filament may be displaced, even if bonded (e.g., by adhesive) in position. As shown in FIG. 20B, this may result in the suture (or some other restraining filament) being displaced proximally.

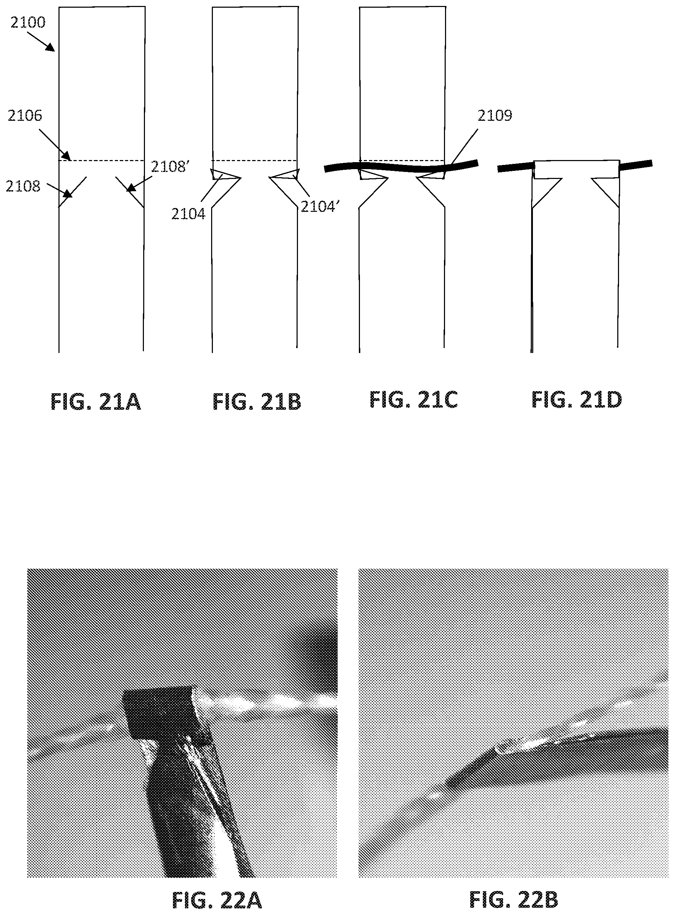

FIGS. 21A-21D illustrate one example of a design configured to form a proximal restraint for a restraining filament out of the tines. In this example, each tine 2100 may be cut or formed to include one or more (e.g., two) projections 2104. In FIG. 21A the tine, which will be folded over at the indicated region 2106 may be cut 2108 to form two projections 2104, 2104' (shown in FIG. 21B) that may form proximal restraints. FIG. 21C shows the placement of a suture forming the restraining filament that may be secured, as shown in FIG. 21D by folding over the distal end of the tine, allowing the bent regions (projections 2104, 2104') to restrain the filaments, as shown.

FIGS. 22D and 22B show side and end views, respectively, of a tine that includes a formed proximal restraint that holds the restraining filament distally, preventing it from migrating proximally and therefore preventing the tines from extending distally out of the mesh forming the walls of the funnel.

In any of the funnels described herein, the funnel may be configured so that it only fully expands when the axial loads are applied, e.g., when pulling the flexible tube (e.g., tractor) proximally to roll into the inversion support catheter. This may allow the funnel to be advanced in smaller vessels before it is actuated.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

In general, any of the apparatuses and methods described herein should be understood to be inclusive, but all or a sub-set of the components and/or steps may alternatively be exclusive, and may be expressed as "consisting of" or alternatively "consisting essentially of" the various components, steps, sub-components or sub-steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure.

## Claims

1.  An apparatus (300) for removing material from a vessel, the apparatus comprising:

    an inversion support comprising a catheter (307), the catheter having an elongate flexible catheter body, the catheter body having a lumen in communication with an open distal end of the catheter body,
    the inversion support further comprising an expandable funnel (308) disposed at a distal end of the catheter body and extending in a proximal to distal axis, wherein a distal end of the funnel (308) defines a distal end opening in communication with an interior of the funnel and the catheter lumen, respectively, and wherein, in an open configuration, an interior wall of the funnel has a wall angle of less than 14 degrees relative to the proximal to distal axis for a majority of a length of the funnel, and includes a first region (350) in which the wall angle is between 14 and 50 degrees with respect to the proximal to distal axis; and
    a tractor comprising a flexible tube (305) that extends distally in an un-inverted configuration along an outer surface of the catheter (307), inverts into the distal end opening of the funnel (308), and extends proximally within the funnel interior and catheter lumen, respectively, in an inverted configuration, wherein the flexible tube (305) is configured to invert by rolling over the distal end opening of the funnel (308) when a first end of the tractor is pulled proximally within the catheter lumen.

2.  The apparatus (330) of claim 1, wherein the funnel (308) comprises a mesh forming the interior wall of the funnel, wherein the mesh inverts at the distal end opening of the funnel and forms an exterior wall of the funnel.

3. The apparatus (300) of claim 2, the exterior wall having a wall angle that is less than 14 degrees with respect to the proximal to distal axis.

4. The apparatus (300) of any one of claims 2-3, wherein the funnel (308) comprises a plurality of longitudinal tines that are continuous with the catheter body proximal of the funnel and disposed between the interior wall and exterior wall.

5. The apparatus (300) of any one of claims 1-4, the interior wall further comprising a second region (351) in which the wall angle is between 14 and 50 degrees with respect to the distal to proximal axis, wherein the second region is separated from the first region (350) by an intermediate region in which the wall angle is less than 14 degrees relative to the proximal to distal axis.

6. The apparatus (300) of any one of claims 1-5, wherein at least one portion of the interior wall having a wall angle of less than 14 degrees relative to the proximal to distal axis has a negative wall angle relative to the proximal to distal axis, such that the interior wall constricts from the proximal to distal direction.

7. The apparatus (300) of any one of claims 1-6, wherein at least a base region (313) of the funnel (308) adjacent to the distal end of the catheter body comprises a plurality of openings configured to allow fluid to pass therethrough.

8. The apparatus (300) of claim 7, wherein the base region (313) comprises a circumferential porous region.

9. The apparatus (300) of any one of claims 1-8, wherein the funnel (308) has a collapsed configuration having a maximum outer diameter of less than 0.3x an outer diameter of the catheter body proximal of the funnel.

10. The apparatus (300) of any one of claims 1-9, wherein the funnel (308) has a minimum outer diameter of greater than 1.5x an outer diameter of the catheter body proximal of the funnel in the open configuration.

11. The apparatus (300) of claim 10, wherein the funnel (308) has an outer diameter of between 2 mm and 26 mm in the open configuration.

12. The apparatus (300) of any one of claims 1-11, wherein the flexible tube (305) comprises a knitted tube.

13. The apparatus (300) of any one of claims 1-12, wherein the funnel (308) is configured to open into the open configuration from a collapsed configuration when the flexible tube (305) is pulled proximally into the catheter lumen and exerts an axial compressive force on the distal end of the funnel.

14. The apparatus (300) of claim 13, wherein, in the open configuration, the funnel (308) is held in a jammed state when the flexible tube (305) is pulled proximally into the catheter lumen, and wherein the funnel has a greater column strength in the jammed state than when the funnel is not in the jammed state.

15. The apparatus (300) of claim 14, wherein in the jammed state, the funnel (308) is configured to withstand greater than 11 Newtons of compressive force without collapsing.

**Patentansprüche**

1. Vorrichtung (300) zur Entfernung von Material aus einem Gefäß, wobei die Vorrichtung aufweist:

eine Inversionsstütze mit einem Katheter (307), wobei der Katheter einen länglichen flexiblen Katheterkörper hat und der Katheterkörper ein Lumen in Kommunikation mit einem offenen distalen Ende des Katheterkörpers hat, wobei die Inversionsstütze ferner einen expandierbaren Trichter (308) aufweist, der an einem distalen Ende des Katheterkörpers angeordnet ist und sich in einer Achse von proximal nach distal erstreckt, wobei ein distales Ende des Trichters (308) eine distale Endöffnung in Kommunikation mit einem Inneren des Trichters und des Katheterlumens definiert und wobei in einer offenen Konfiguration eine Innenwand des Trichters einen Wandwinkel unter 14 Grad relativ zur Achse von proximal nach distal über einen Großteil einer Länge des Trichters hat und einen ersten Bereich (350) aufweist, in dem der Wandwinkel zwischen 14 und 50 Grad in Bezug auf die Achse von proximal nach distal beträgt; und
einen Traktor mit einem flexiblen Schlauch (305), der sich in einer nicht invertierten Konfiguration entlang einer Außenfläche des Katheters (307) distal erstreckt, in die distale Endöffnung des Trichters (308) invertiert und sich im Trichterinneren und Katheterlumen in einer invertierten Konfiguration proximal erstreckt, wobei der flexible Schlauch (305) so konfiguriert ist, dass er durch Rollen über die distale Endöffnung des Trichters (308) invertiert, wenn ein erstes Ende des Traktors im Katheterlumen proximal gezogen wird.

2. Vorrichtung (330) nach Anspruch 1, wobei der Trichter (308) ein Maschenmaterial aufweist, das die Innenwand des Trichters bildet, wobei das Maschenmaterial an der distalen Endöffnung des Trichters invertiert und eine Außenwand des Trichters bildet.

3. Vorrichtung (300) nach Anspruch 2, wobei die Außenwand einen Wandwinkel hat, der unter 14 Grad in Bezug auf die Achse von proximal nach distal beträgt.

4. Vorrichtung (300) nach einem der Ansprüche 2 bis 3, wobei der Trichter (308) mehrere Längszinken aufweist, die mit dem Katheterkörper proximal vom Trichter kontinuierlich und zwischen der Innenwand und Außenwand angeordnet sind.

5. Vorrichtung (300) nach einem der Ansprüche 1 bis 4, wobei die Innenwand ferner einen zweiten Bereich (351) aufweist, in dem der Wandwinkel zwischen 14 und 50 Grad in Bezug auf die Achse von distal nach proximal beträgt, wobei der zweite Bereich vom ersten Bereich (350) durch einen Zwischenbereich getrennt ist, in dem der Wandwinkel unter 14 Grad relativ zur Achse von proximal nach distal beträgt.

6. Vorrichtung (300) nach einem der Ansprüche 1 bis 5, wobei zumindest ein Abschnitt der Innenwand mit einem Wandwinkel unter 14 Grad relativ zur Achse von proximal nach distal einen negativen Wandwinkel relativ zur Achse von proximal nach distal hat, so dass sich die Innenwand in der Richtung von proximal nach distal verengt.

7. Vorrichtung (300) nach einem der Ansprüche 1 bis 6, wobei mindestens ein Basisbereich (313) des Trichters (308) benachbart zum distalen Ende des Katheterkörpers mehrere Öffnungen aufweist, die so konfiguriert sind, dass ein Fluid hindurchfließen kann.

8. Vorrichtung (300) nach Anspruch 7, wobei der Basisbereich (313) einen porösen Umfangsbereich aufweist.

9. Vorrichtung (300) nach einem der Ansprüche 1 bis 8, wobei der Trichter (308) eine kollabierte Konfiguration mit einem maximalen Außendurchmesser unter dem 0,3-Fachen eines Außendurchmessers des Katheterkörpers proximal zum Trichter hat.

10. Vorrichtung (300) nach einem der Ansprüche 1 bis 9, wobei der Trichter (308) einen minimalen Außendurchmesser über dem 1,5-Fachen eines Außendurchmessers des Katheterkörpers proximal zum Trichter in der offenen Konfiguration hat.

11. Vorrichtung (300) nach Anspruch 10, wobei der Trichter (308) einen Außendurchmesser zwischen 2 mm und 26 mm in der offenen Konfiguration hat.

12. Vorrichtung (300) nach einem der Ansprüche 1 bis 11, wobei der flexible Schlauch (305) einen Strickschlauch aufweist.

13. Vorrichtung (300) nach einem der Ansprüche 1 bis 12, wobei der Trichter (308) so konfiguriert ist, dass er sich in die offene Konfiguration aus einer kollabierten Konfiguration öffnet, wenn der flexible Schlauch (305) proximal in das Katheterlumen gezogen wird und eine Axialdruckkraft auf das distale Ende des Trichters ausübt.

14. Vorrichtung (300) nach Anspruch 13, wobei in der offenen Konfiguration der Trichter (308) in einem festsitzenden Zustand gehalten wird, wenn der flexible Schlauch (305) proximal in das Katheterlumen gezogen wird, und wobei der Trichter eine größere Knickfestigkeit im festsitzenden Zustand als dann hat, wenn sich der Trichter nicht im festsitzenden Zustand befindet.

15. Vorrichtung (300) nach Anspruch 14, wobei im festsitzenden Zustand der Trichter (308) so konfiguriert ist, dass er über 11 Newton Druckkraft ohne Kollabieren widersteht.

**Revendications**

1. Appareil (300) pour retirer un matériau d'un vaisseau, l'appareil comprenant :

un support d'inversion comprenant un cathéter (307), le cathéter ayant un corps de cathéter flexible allongé, le corps de cathéter ayant une lumière en communication avec une extrémité distale ouverte du corps de cathéter, le support d'inversion comprenant en outre un entonnoir expansible (308) disposé au niveau de l'extrémité distale du corps de cathéter et s'étendant dans un axe proximal à distal, dans lequel une extrémité distale de l'entonnoir (308) définit une ouverture d'extrémité distale respectivement en communication avec un intérieur de l'entonnoir et la lumière de cathéter, et dans lequel, dans une configuration ouverte, une paroi intérieure de l'entonnoir a un angle de paroi inférieur à 14 degrés par rapport à l'axe proximal à distal sur une majeure partie d'une longueur de l'entonnoir, et comprend une première région (350) dans laquelle l'angle de paroi est compris entre 14 et 50 degrés par rapport à l'axe proximal à distal ; et
un dispositif de traction comprenant un tube flexible (305) qui s'étend de manière distale dans une configuration non inversée le long d'une surface externe du cathéter (307), s'inverse dans l'ouverture d'extrémité distale de l'entonnoir (308) et s'étend de manière proximale dans l'intérieur de l'entonnoir et la lumière de cathéter, respectivement, dans une configuration inversée, dans lequel le tube flexible (305) est configuré pour s'inverser par enroulement sur l'ouverture d'extrémité distale de l'entonnoir (308), lorsqu'une première extrémité du dispositif de traction est tirée de manière proximale à l'intérieur de la lumière de cathéter.

2. Appareil (330) selon la revendication 1, dans lequel l'entonnoir (308) comprend une maille formant la paroi intérieure de l'entonnoir, dans lequel la maille s'inverse au niveau de l'ouverture d'extrémité distale de l'entonnoir et forme une paroi extérieure de l'entonnoir.

3. Appareil (300) selon la revendication 2, la paroi extérieure ayant un angle de paroi qui est inférieur à 14 degrés par rapport à l'axe proximal à distal.

4. Appareil (300) selon l'une quelconque des revendications 2 à 3, dans lequel l'entonnoir (308) comprend une pluralité de dents longitudinales qui sont continues avec le corps de cathéter à proximité de l'entonnoir et disposées entre la paroi intérieure et la paroi extérieure.

5. Appareil (300) selon l'une quelconque des revendications 1 à 4, la paroi intérieure comprenant en outre une deuxième région (351) dans lequel l'angle de paroi est compris entre 14 et 50 degrés par rapport à l'axe distal à proximal, dans lequel la deuxième région est séparée de la première région (350) par une région intermédiaire dans laquelle l'angle de paroi est inférieur à 14 degrés par rapport à l'axe proximal à distal.

6. Appareil (300) selon l'une quelconque des revendications 1 à 5, dans lequel au moins une partie de la paroi intérieure ayant un angle de paroi inférieur à 14 degrés par rapport à l'axe proximal à distal a un angle de paroi négatif par rapport à l'axe proximal à distal, de sorte que la paroi intérieure se contracte de la direction proximale à distale.

7. Appareil (300) selon l'une quelconque des revendications 1 à 6, dans lequel au moins une région de base (313) de l'entonnoir (308) adjacent à l'extrémité distale du corps de cathéter comprend une pluralité d'ouvertures configurées pour permettre le passage du fluide à travers ces dernières.

8. Appareil (300) selon la revendication 7, dans lequel la région de base (313) comprend une région poreuse circonférentielle.

9. Appareil (300) selon l'une quelconque des revendications 1 à 8, dans lequel l'entonnoir (308) a une configuration repliée ayant un diamètre externe maximum inférieur à 0,3 fois un diamètre externe du corps de cathéter à proximité de l'entonnoir.

10. Appareil (300) selon l'une quelconque des revendications 1 à 9, dans lequel l'entonnoir (308) a un diamètre externe minimum supérieur à 1,5 fois un diamètre externe du corps de cathéter à proximité de l'entonnoir dans la configuration ouverte.

11. Appareil (300) selon la revendication 10, dans lequel l'entonnoir (308) a un diamètre externe compris entre 2 mm et 26 mm dans la configuration ouverte.

**12.** Appareil (300) selon l'une quelconque des revendications 1 à 11, dans lequel le tube flexible (305) comprend un tube tricoté.

**13.** Appareil (300) selon l'une quelconque des revendications 1 à 12, dans lequel l'entonnoir (308) est configuré pour s'ouvrir dans la configuration ouverte à partir d'une configuration repliée lorsque le tube flexible (305) est tiré de manière proximale dans la lumière de cathéter et exerce une force de compression axiale sur l'extrémité distale de l'entonnoir.

**14.** Appareil (300) selon la revendication 13, dans lequel, dans la configuration ouverte, l'entonnoir (308) est maintenu dans un état bloqué lorsque le tube flexible (305) est tiré, de manière proximale, dans la lumière de cathéter, et dans lequel l'entonnoir a une plus grande résistance colonnaire à l'état bloqué que lorsque l'entonnoir n'est pas à l'état bloqué.

**15.** Appareil (300) selon la revendication 14, dans lequel, à l'état bloqué, l'entonnoir (308) est configuré pour résister à plus de 11 Newtons de force de compression sans affaissement.

FIG. 1A

FIG. 1B

FIG. 1C

FIG. 2A

FIG. 2B

FIG. 1D

344

FIG. 3A

330

FIG. 3B

404

406

FIG. 4A

FIG. 4B

FIG. 4C

| Number of loops/turn | French | Circumference of inversion support catheter (mm) | Locking force | wire thickness (inches) | wire thickness (mm) | (number of loops)$^2$*wire diameter)/catheter circumference |
|---|---|---|---|---|---|---|
| 16 | 9 F | 9.42 | 25 | 0.0022 | 0.05588 | 1.519 |
| 16 | 9 F | 9.42 | 25 | 0.0022 | 0.05588 | 1.519 |
| 21 | 5 F | 5.24 | u | 0.001 | 0.0254 | 2.138 |
| 18 | 9 F | 9.42 | u | 0.0022 | 0.05588 | 1.922 |
| 16 | 9 F | 9.42 | u | 0.0025 | 0.0635 | 1.726 |
| 21 | 9 F | 9.42 | u | 0.0022 | 0.05588 | 2.616 |
| 22 | 5 F | 5.24 | 20 | 0.0012 | 0.03048 | 2.815 |
| 24 | 9 F | 9.42 | 300 | 0.002 | 0.0508 | 3.106 |
| 24 | 5 F | 5.24 | a | 0.0012 | 0.03048 | 3.350 |
| 24 | 9 F | 9.42 | 556 | 0.0022 | 0.05588 | 3.417 |
| 27 | 5 F | 5.24 | a | 0.0012 | 0.03048 | 4.240 |
| 28 | 5F | 5.24 | a | 0.0012 | 0.03048 | 4.560 |
| 28 | 9 F | 9.42 | a | 0.0022 | 0.05588 | 4.651 |
| 30 | 5 F | 5.24 | a | 0.0012 | 0.03048 | 5.235 |
| 30 | 9 F | 9.42 | a | 0.0022 | 0.05588 | 5.339 |
| 32 | 9 F | 9.42 | a | 0.0022 | 0.05588 | 6.074 |
| 34 | 5 F | 5.24 | 120 | 0.0012 | 0.03048 | 6.724 |
| 32 | 5 F | 5.24 | a | 0.0022 | 0.05588 | 10.920 |

FIG. 5

FIG. 6

10mm Vessel

Clot

8F/3mm Catheter

601

FIG. 7A

309

307

303

300

305

313

308

315

FIG. 7B

309

300

307

303

305

313

351

350

308

315

FIG. 8C

FIG. 8B

FIG. 8A

FIG. 9A                    FIG. 9B

1007

1013

**FIG. 10A**

1007

1023

1013

**FIG. 10B**

1007

1013

**FIG. 10C**

1107
CATHETER

1105
BRAID IS
ATTACHED TO
CATHETER

1111
FINGERS* ON CATHETER

1109
IN SIDE/OUTSIDE
LOCKED TOGETHER

1118

**FIG. 11A**

**FIG. 11B**

1105

1109

1131

**FIG. 11C**

**FIG. 11D**

FIG. 12

FIG. 13

FIG. 14

**FIG. 15A**

**FIG. 15B**

FIG. 16A

FIG. 16B

**FIG. 16C**

**FIG. 16D**

1700

1709

**FIG. 17**

1815

1816

**FIG. 18A**

$\alpha_1$  $\beta_1$

$\alpha_2$

$\beta_2$

30 deg

-15 deg

35 deg

-2 deg

**FIG. 18B**

1900

1909

**FIG. 19**

2005

2007

**FIG. 20A**

2005      2007

**FIG. 20B**

**FIG. 21A**          **FIG. 21B**          **FIG. 21C**          **FIG. 21D**

**FIG. 22A**                    **FIG. 22B**

**REFERENCES CITED IN THE DESCRIPTION**

**Patent documents cited in the description**

- US 10271864 B
- US 20170303948
- US 20190117214 A
- US 20180042626 A
- US 20180042624 A
- US 566393
- US 20190336148